# EUROPÄISCHE PATENTSCHRIFT

(11) **EP 0 970 086 B1**
(45) Veröffentlichungstag und Bekanntmachung des Hinweises auf die Patenterteilung: **10.12.2003**
(21) Anmeldenummer: 98913731.0
(22) Anmeldetag: 18.03.1998
(51) Int. Cl.: C07D 495/22, C07D 471/10, C07D 471/22, H05B 33/14, H01L 51/30

(54) **SPIROVERBINDUNGEN UND DEREN VERWENDUNG**
SPIRO COMPOUNDS AND THE USE THEREOF
COMPOSES SPIRO ET LEUR UTILISATION

(30) Priorität: 20.03.1997 DE 19711568
(43) Veröffentlichungstag der Anmeldung: 12.01.2000
(73) Patentinhaber: Covion Organic Semiconductors GmbH, 65926 Frankfurt (DE)
(72) Erfinder: SALBECK, Josef, D-65779 Kelkheim (DE); LUPO, Donald, D-60316 Frankfurt (DE)
(74) Vertreter: Luderschmidt, Schüler & Partner GbR
(86) Internationale Anmeldenummer: EP9801589
(87) Internationale Veröffentlichungsnummer: WO98042715

(56) Entgegenhaltungen:
- EP-A- 0 333 641
- EP-A- 0 676 461
- EP-A- 0 718 858
- WO-A-97/10617
- CHEMICAL ABSTRACTS, vol. 124, no. 3, 1996 Columbus, Ohio, US; abstract no. 29632x, PIOTROWIAK: "Novel ligands for electron transfer transition metal complexes" Seite 965; XP002073522 & BULL. POL. ACAD. SCI., CHEM. 1994, Bd. 42, Nr. 4, 1995, Seiten 445-453,
- H. WYNBERG ET AL.: "The synthesis and spectra of some hererocyclic conjugated spiranes" RECUEIL DES TRAVAUX CHIMIQUES DES PAYS-BAS., Bd. 89, 1970, Seite 545 XP002073521 AMSTERDAM NL

## Beschreibung

Aufgrund des weltweit steigenden Bedarfs an elektrischer Energie und der begrenzten Vorräte an Kohle, Öl und Gas, die bei ihrer Verbrennung zudem das Treibhausgas CO₂ freisetzen, hat die Erzeugung von elektrischem Strom aus Sonnenlicht in den letzten Jahre erhöhtes Interesse gefunden.

In der EP-A 0 333 641 ist eine photoelektrochemische Zelle beschrieben, die dadurch gekennzeichnet ist, daß sie einen nanoporösen, d. h. eine extrem aufgerauhte und damit vergrößerte Oberfläche aufweisenden Metalloxid-Halbleiter enthält. Der Ladungstransport zwischen Halbleiter/Chromophor-Schicht und Gegenelektrode erfolgt in dieser Zelle durch eine Elektrolytlösung.
Obwohl mit solchen Zellen gute Ergebnisse erzielt werden, ist das Eigenschaftsprofil einer solchen Vorrichtung noch deutlich verbesserungsfähig.

Aus der EP-A 0 718 858 ist eine solche Zelle mit einem flüssigkristallinen Ladungstransportmaterial anstelle eines Elektrolyten bekannt. Die erreichten scheinbaren Quantenausbeuten sind aber noch verbesserungsbedürftig.

Es wurde nun überraschend gefunden, daß sich bestimmte Spiroverbindungen in hervorragender Weise als Ladungstransportmaterial für photovoltaische Zellen eignen.

Einzelne strukturell andersartige Spirobifluorenderivate sind beispielsweise in US-A 5,026,894, J.M. Tour et al. J. Am. Chem. Soc. 112 (1990) 5662 und J.M. Tour et al. Polym. Prepr. (1990) 408 als Verknüpfungselemente für polymere, organische Halbleiter beschrieben und als Materialien für molekulare Elektronik vorgeschlagen.

In der EP-A 0 676 461 ist die Verwendung von Spiroverbindungen der folgenden allgemeinen Formel 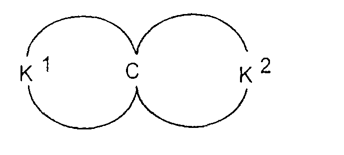 wobei
K¹ und K² unabhängig voneinander konjugierte Systeme bedeuten,
in Elektrolumineszenzvorrichtungen, beschreiben.

Eine Anwendung in photovoltaischen Zellen läßt sich daraus nicht herleiten.

Ein Gegenstand der Erfindung sind daher Spiroverbindungen der allgemeinen Formel (I), 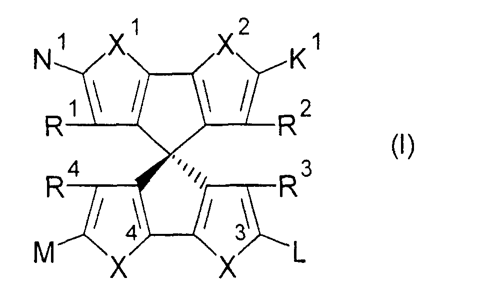 wobei die Symbole folgende Bedeutungen haben:
- X¹, X², X³, X⁴: sind gleich oder verschieden -S-, -O-, -NR⁵-, -CR⁵=N-, -CR⁵=CH-, mit der Maßgabe, daß mindestens eine der Gruppen X¹⁻⁴ von -CR⁵=CH- verschieden ist;
- K¹, L, M, N¹, R¹, R², R³, R⁴: sind gleich oder verschieden und bedeuten
a) Wasserstoff, -NO₂, -CN, -F oder -Cl,
b) ein geradkettiger oder verzweigter Alkylrest mit 1 bis 20 C-Atomen, wobei
   b1 ) eine oder mehrere nicht benachbarte CH₂-Gruppen durch -O-, -S-, - CO-O-, -O-CO-, -O-CO-O-, NR⁵ oder -Si(CH₃)₂- ersetzt sein können und/oder
   b2) eine oder mehrere CH₂-Gruppen durch -CH=CH-, -C≡C-, 1,4-Phenylen, 1,4-Cyclohexylen oder 1,3-Cyclopentylen ersetzt sein können und/oder
   b3) ein oder mehrere H-Atome durch F und/oder Cl ersetzt sein können und/oder
c) eine der folgenden Gruppen: 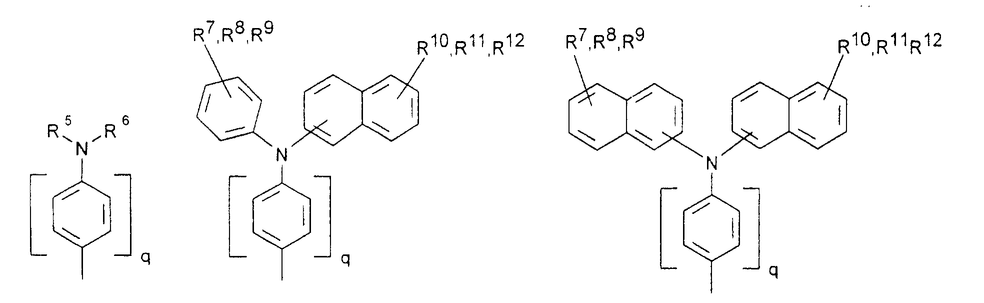 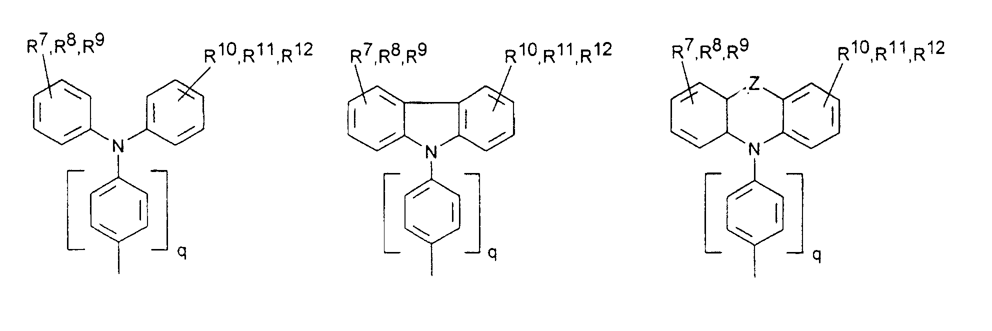 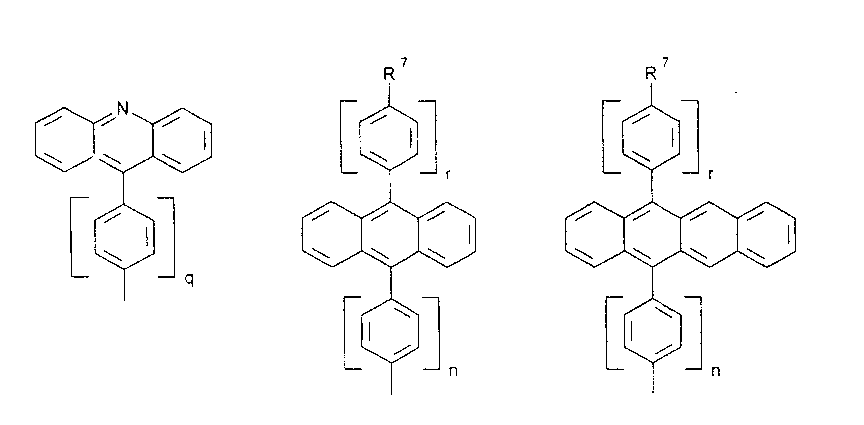 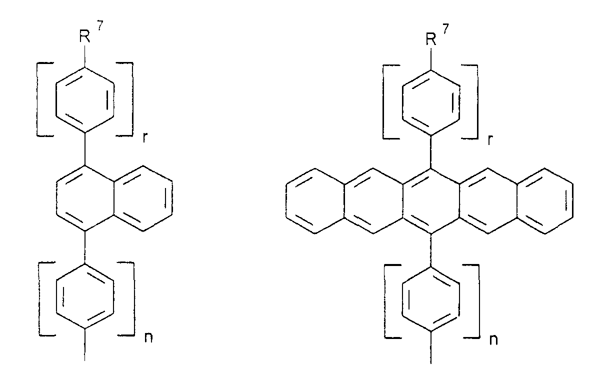 oder 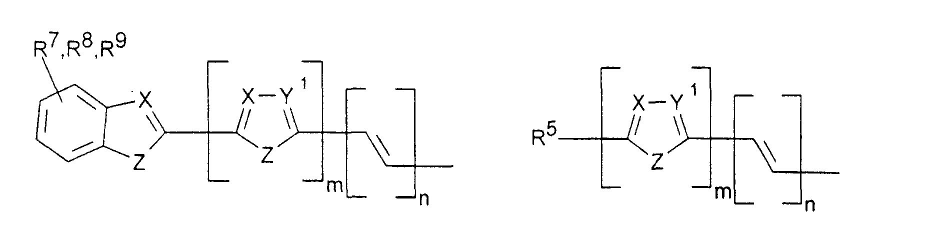 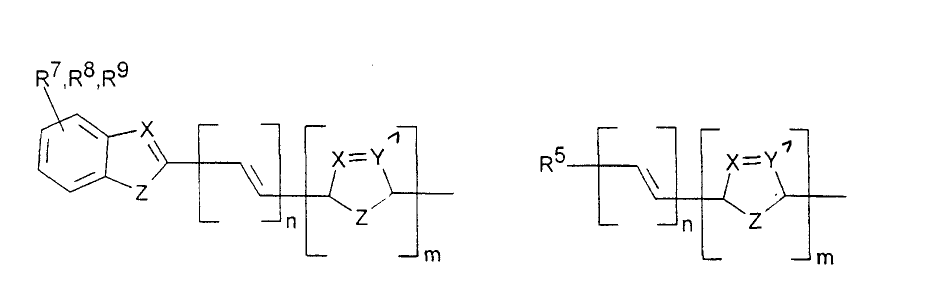
- X, Y¹: sind jeweils gleich oder verschieden =CR⁷- oder =N-;
- Z: ist -O-, -S-, -NR⁵-;
- R⁵, R⁶: sind jeweils gleich oder verschieden
a) Wasserstoff
b) ein geradkettiger oder verzweigter Alkylrest mit 1 bis 20 C-Atomen, wobei
   b1 ) eine oder mehrere nicht benachbarte und nicht an Stickstoff gebundene CH₂-Gruppen durch -O-, -S-, -CO-O-, -O-CO-, -O-CO-O- oder -Si(CH₃)₂ ersetzt sein können und/oder
   b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
   b3) ein oder mehrere H-Atome durch F und/oder Cl ersetzt sein können und/oder
   b4) R⁵ und R⁶ zusammen auch einen Ring bilden können;
c) Phenyl, Biphenyl, 1-Naphthyl, 2-Naphthyl, 2-Thienyl, 2-Furanyl;
- R⁷, R⁸, R⁹, R¹⁰, R¹¹, R¹²: sind gleich oder verschieden
a) Wasserstoff, -CN, -F, NO₂ oder -Cl
b) ein geradkettiger oder verzweigter Alkylrest mit 1 bis 20 C-Atomen, wobei
   b1) eine oder mehrere nicht benachbarte CH₂-Gruppen durch -O-, - S-, -CO-O-, -O-CO-, -O-CO-O-, -NR⁵ oder -Si(CH₃)₂- ersetzt sein können und/oder
   b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
   b3) ein oder mehrere H-Atome durch F und/oder Cl ersetzt sein können;
c) Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl,
- m, n, p, q, r: sind jeweils gleich oder verschieden 0, 1, 2, 3, 4, 5 oder 6, vorzugsweise 0, 1, 2, 3, 4, 5 oder 6, vorzugsweise 0, 1, 2, 3, 4, besonders bevorzugt 0, 1, 2 oder 3,
ausgenommen die Verbindungen der Formel 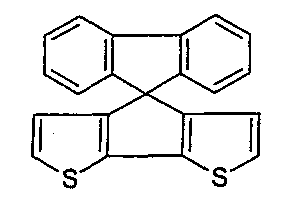 sowie 4,5-Diazospirobifluoren und 4,4',5,5'-Tetraazosptrobifluoren.

Die Verbindungen der Formel (I) sind vorzugsweise amorph und zeichnen sich durch hohe Glasübergangstemperaturen aus.

Bevorzugt sind Verbindungen der Formel (I) bei denen
a) X¹⁻⁴ identisch sind oder
b) X¹ gleich X² und X³ gleich X⁴ ist.

Besonders bevorzugt sind folgende Verbindungen der Formel (I):
a) 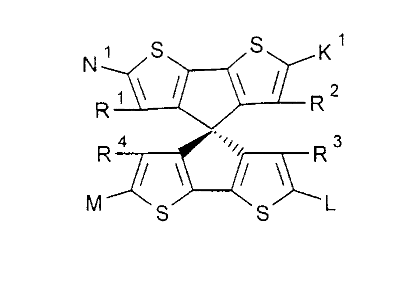
b) 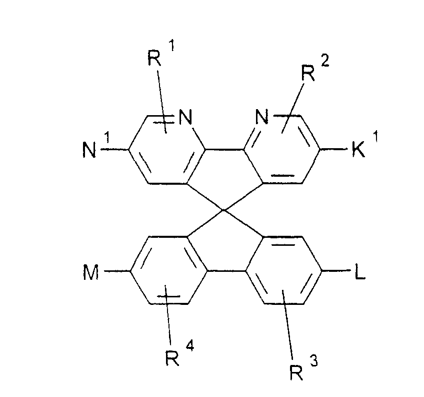
c)

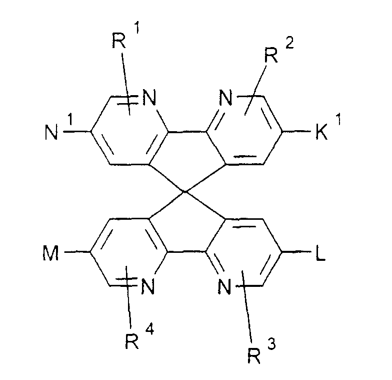

Ganz besonders bevorzugt sind Spirofluorenderivate der Fomeln (I) a-c
wobei die Symbole folgende Bedeutungen haben:
I.a) K¹ = L = M = N¹ und ist aus der Gruppe: 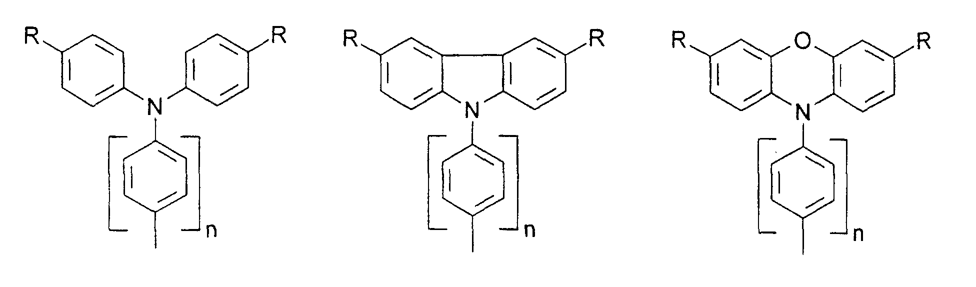 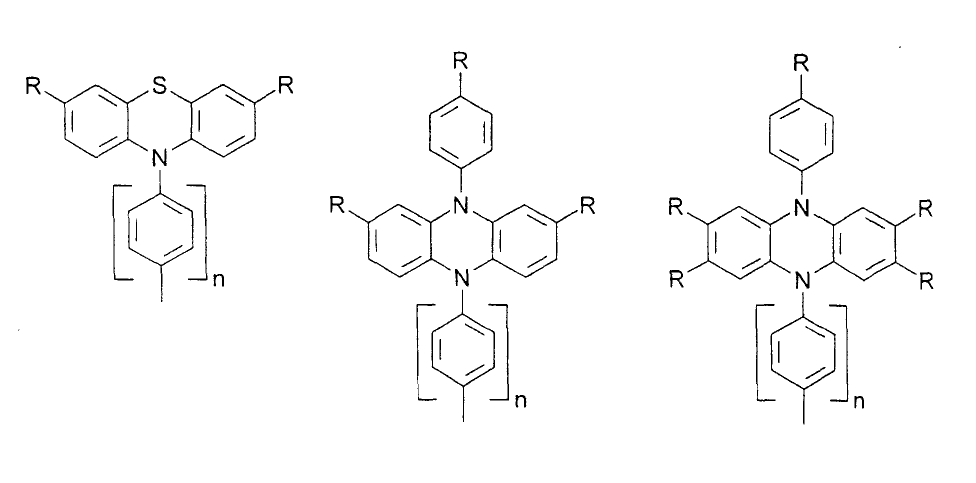 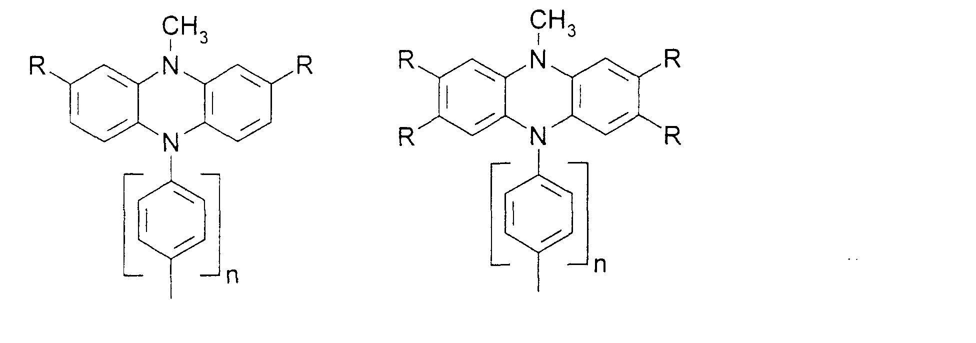 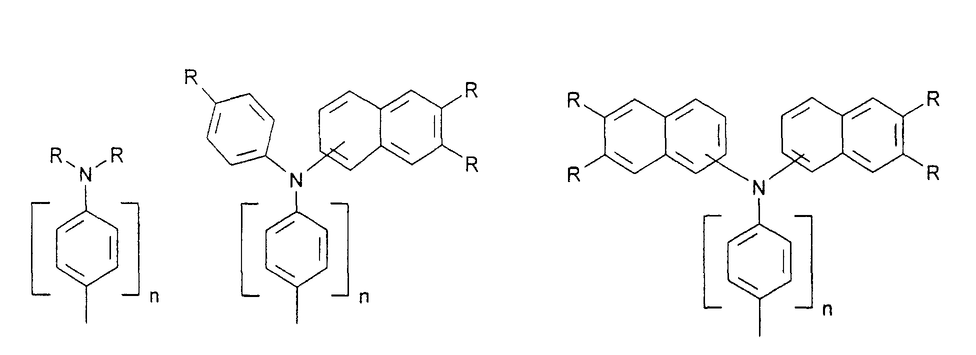 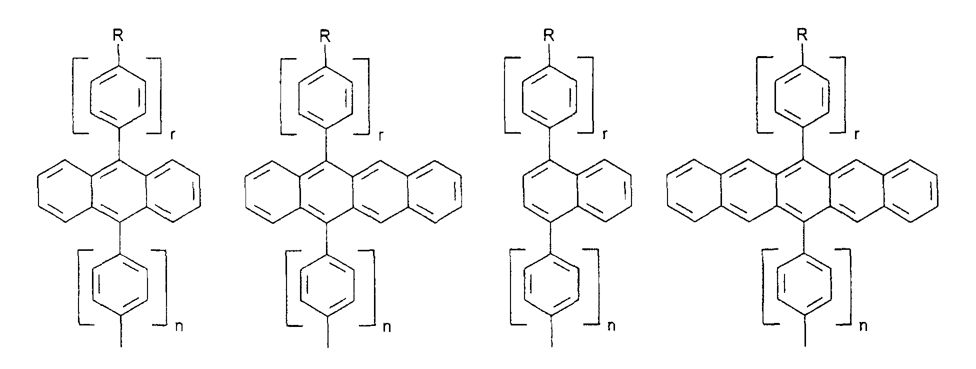
   - R: gleich oder verschieden H, Alkyl, -O-Alkyl, -S-Alkyl, mit jeweils 1 bis 20 C-Atomen, vorzugsweise 1 bis 4 C-Atomen, Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl, CN, NR₂, wobei -O-Alkyl/Aryl, -S-Alkyl/Aryl, CN, NR₂ nicht an Stickstoff gebunden sein darf;
   - n: = 0,1,2,3,4.
   und R¹, R², R³, R⁴ sind gleich oder verschieden und aus der Gruppe
   H, COOR, CH₂OR, 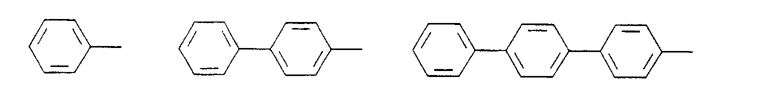 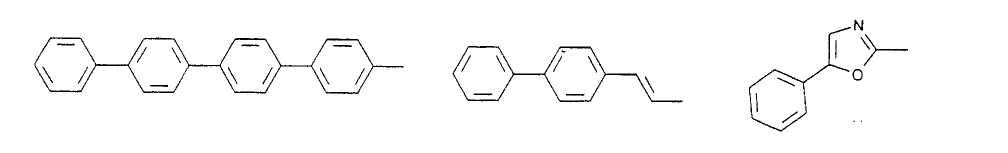 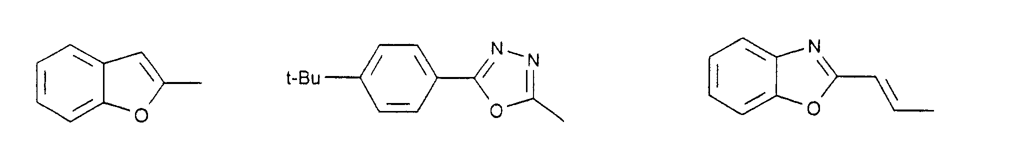 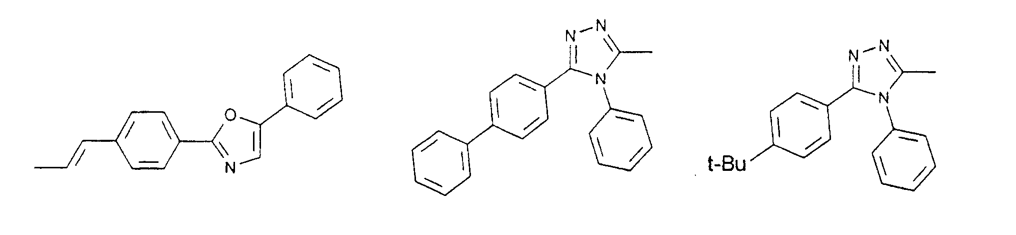 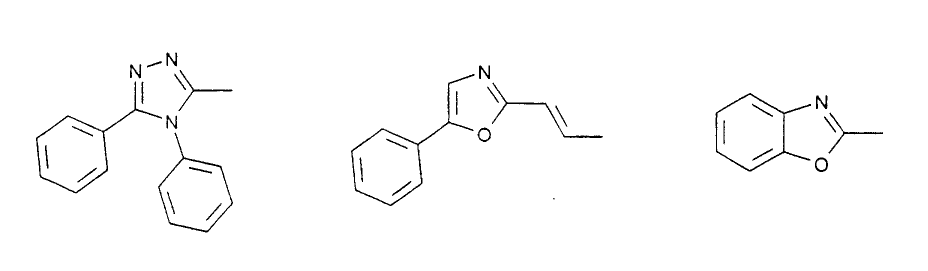 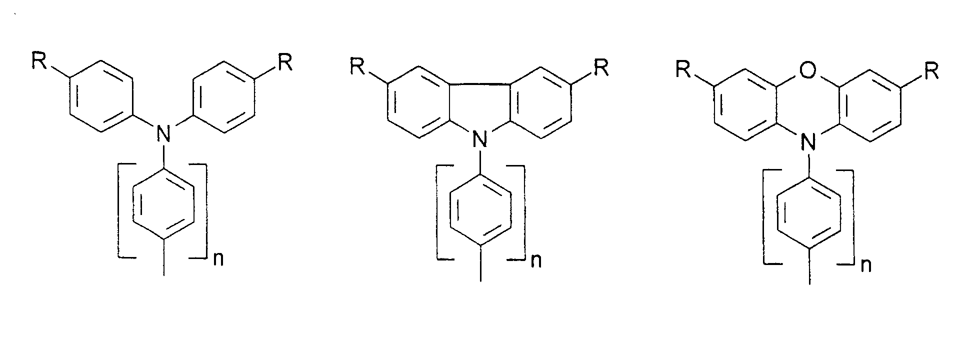 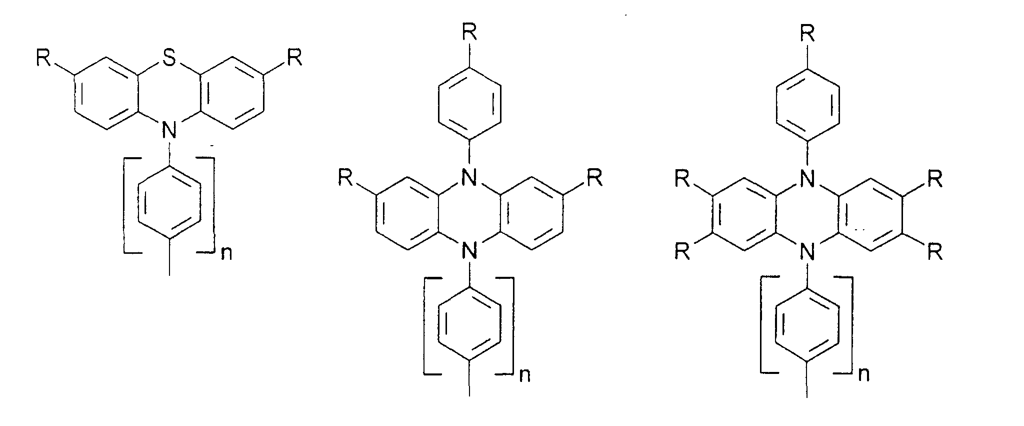 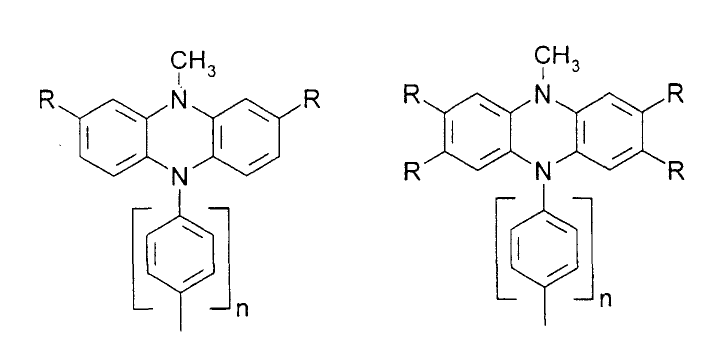 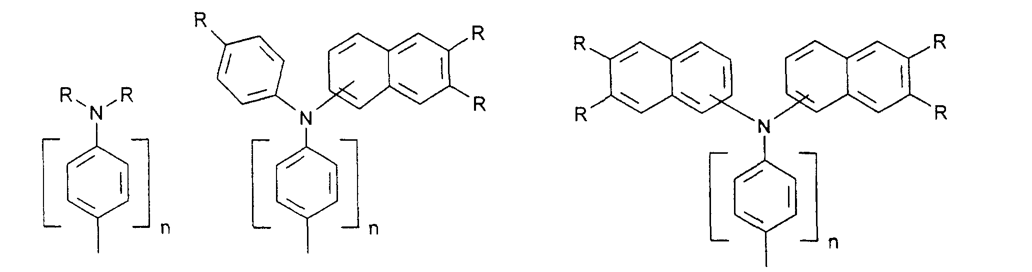 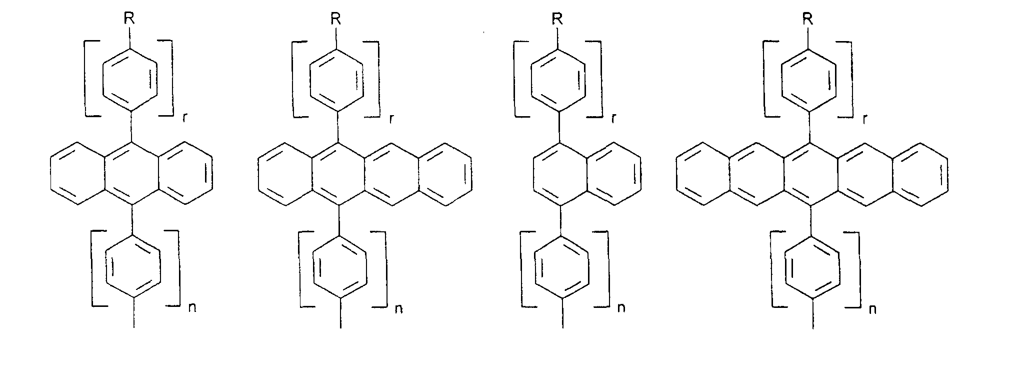 wobei die Symbole und Indizes die oben angegebenen Bedeutungen haben und vorzugsweise mindestens zwei der Reste R¹⁻⁴ H sind.
I.b) K¹ = N¹ und ist aus der Gruppe 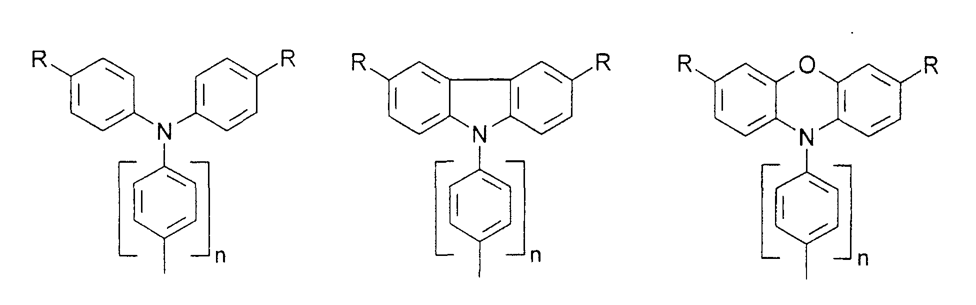 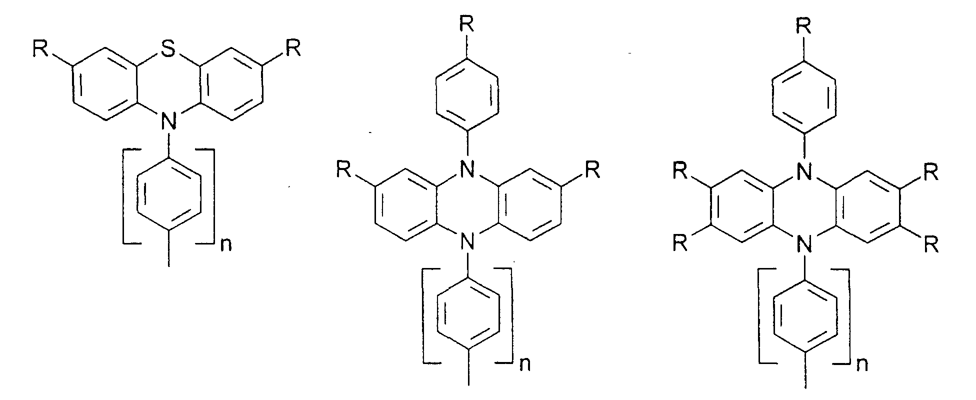 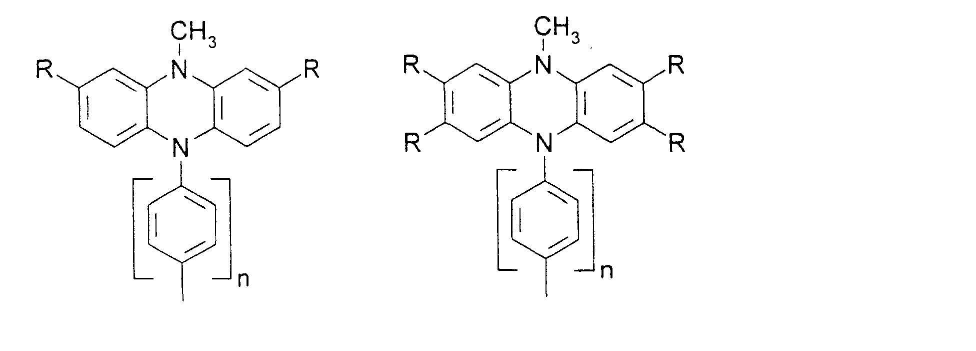 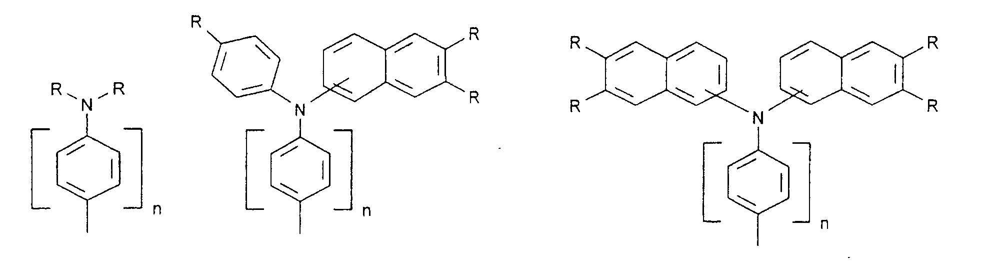 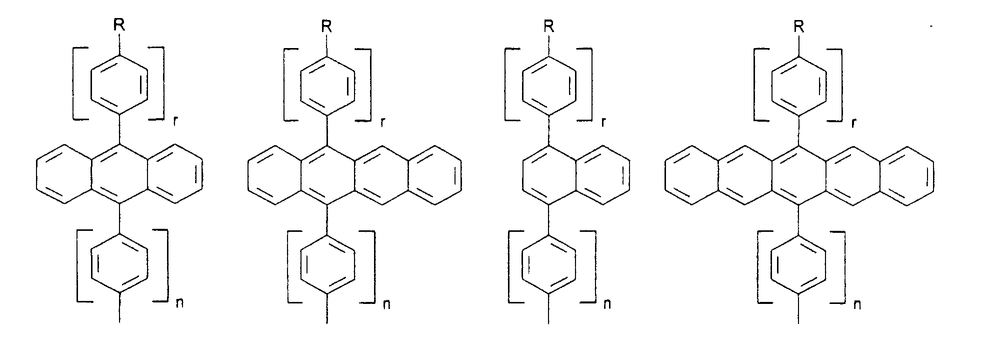 und L = M und ist aus der Gruppe
   H, COOR, CH₂OR, 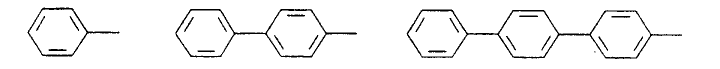 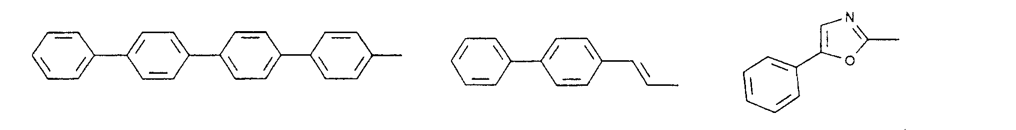 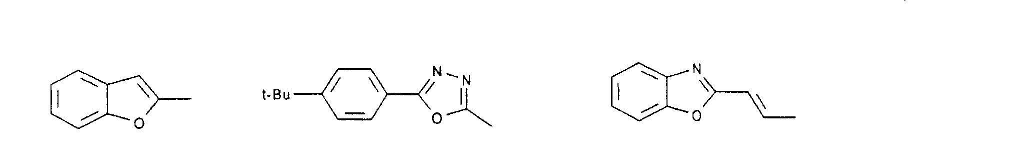 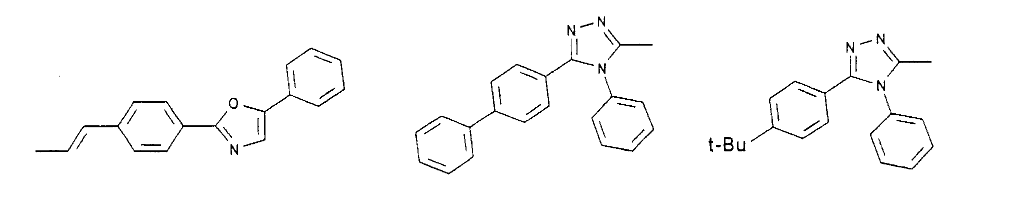 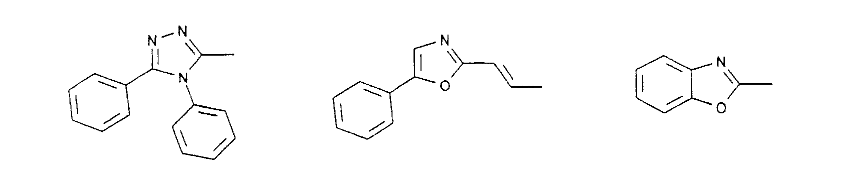 und R¹, R², R³, R⁴ sind gleich oder verschieden aus der Gruppe 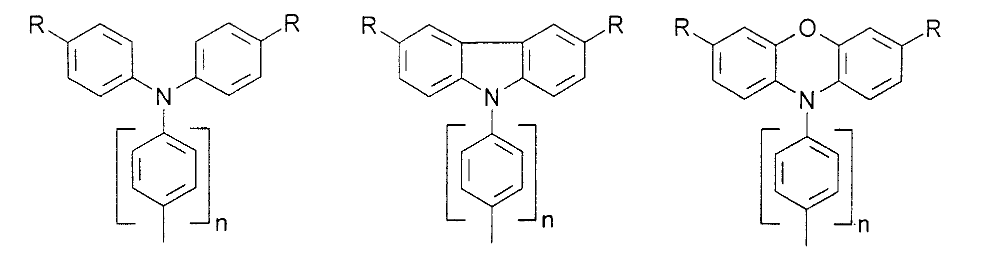 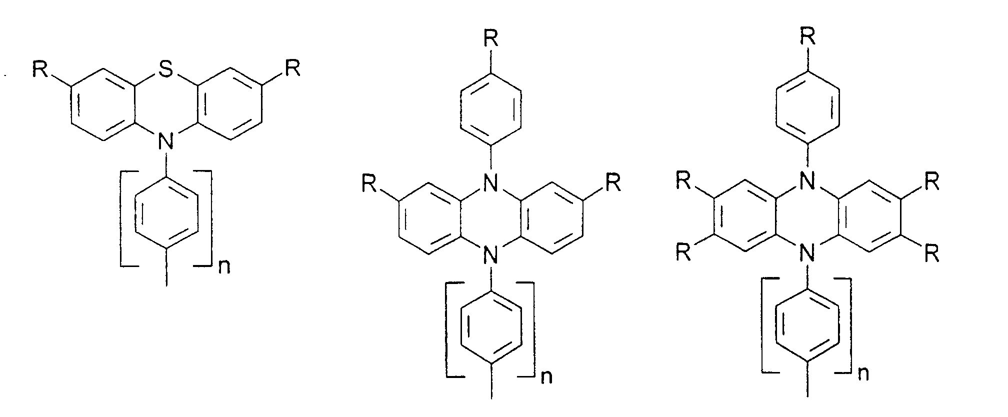 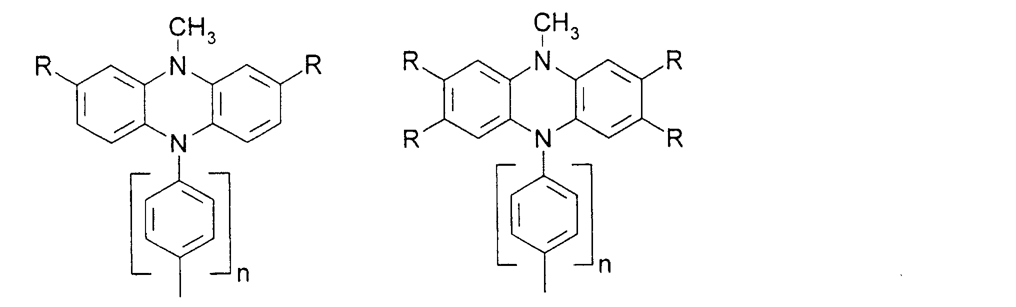 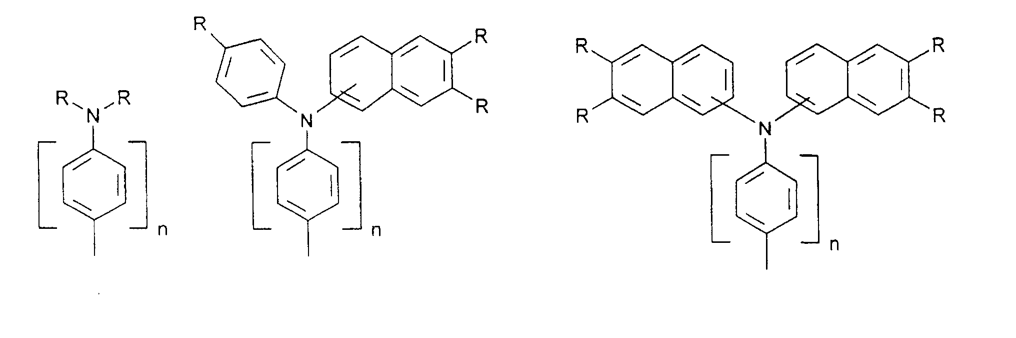 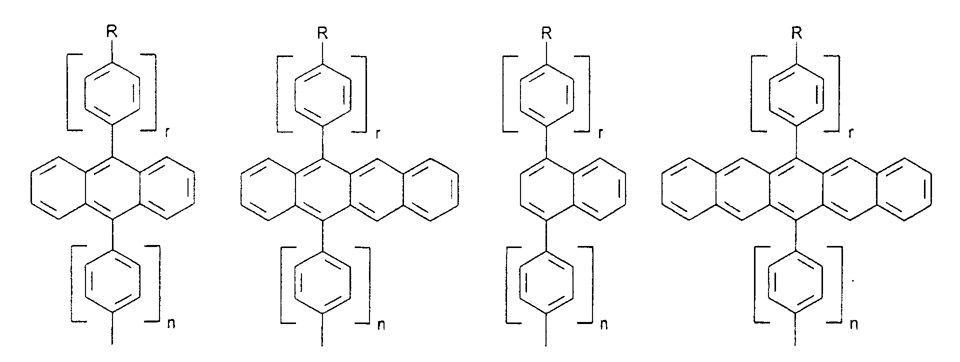 H, COOR, CH₂OR, 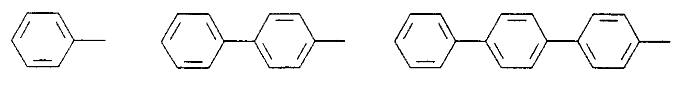 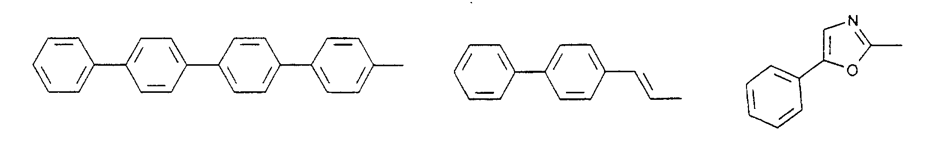 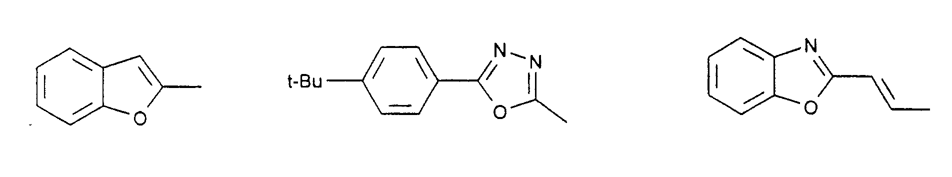 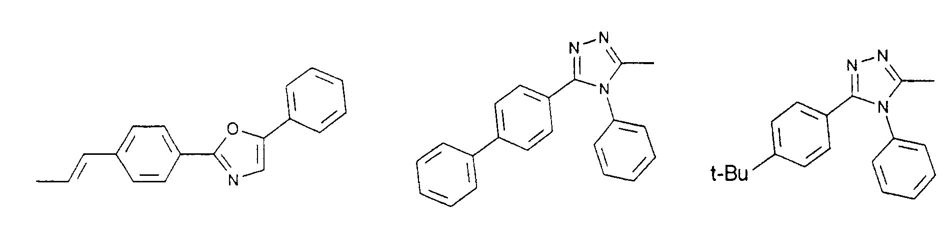 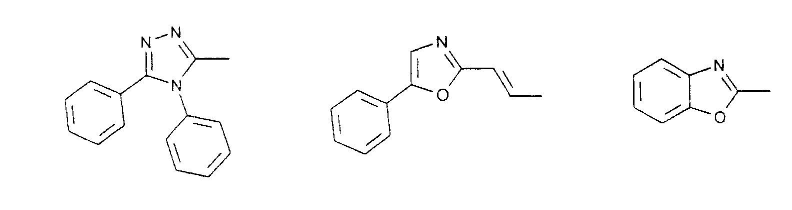 wobei die Symbole und Indizes die oben angegebenen Bedeutungen haben und wobei vorzugsweise mindestens zwei der Reste R¹⁻⁴ H sind.
Ic) K¹ = M und ist aus der Gruppe 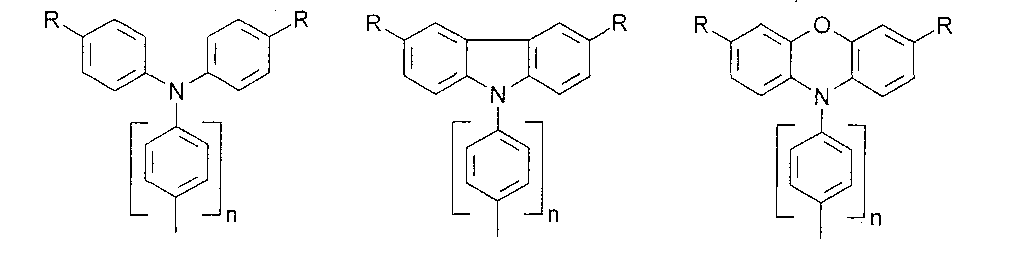 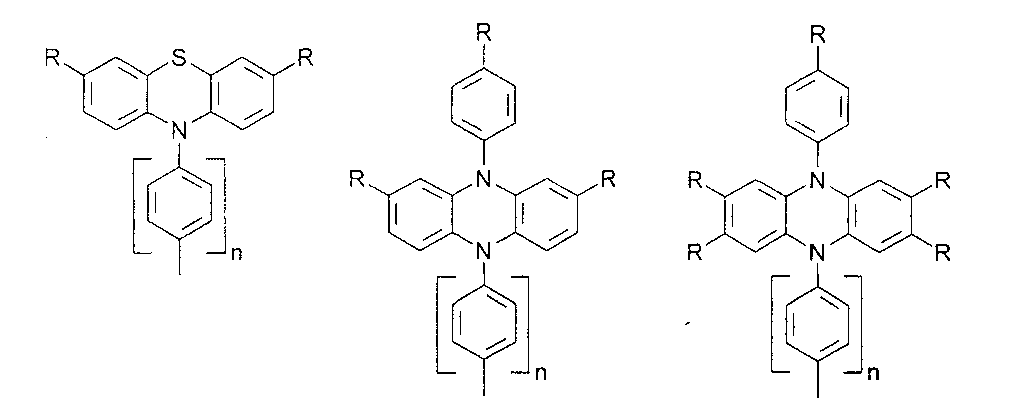 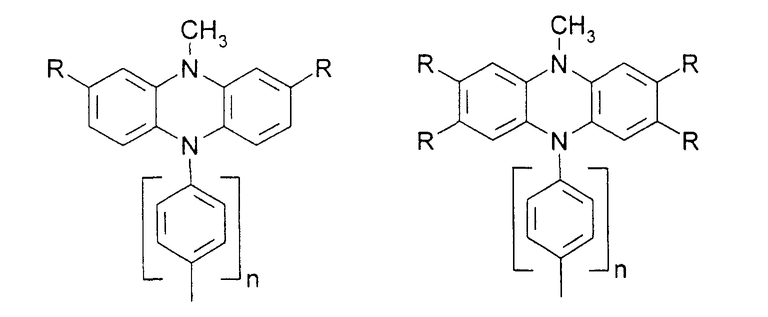 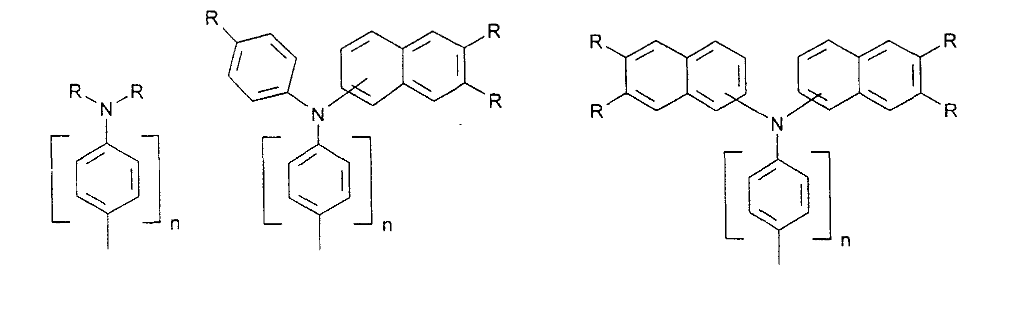 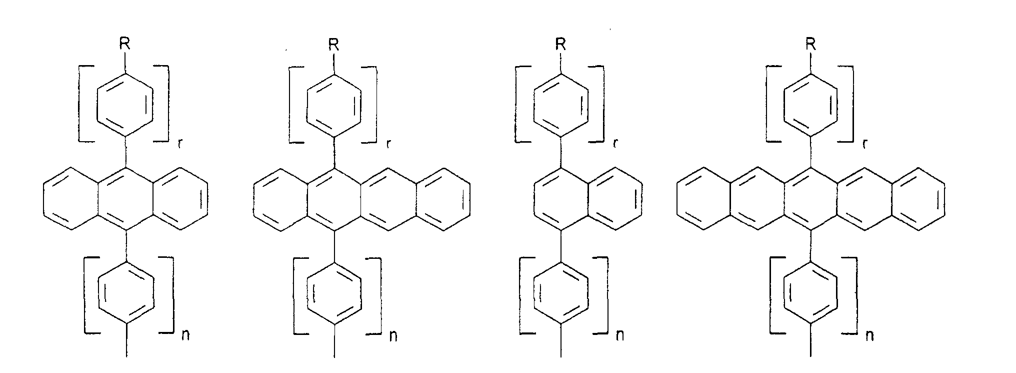 und M = N¹ ist und aus der Gruppe
   H, COOR, CH₂OR, 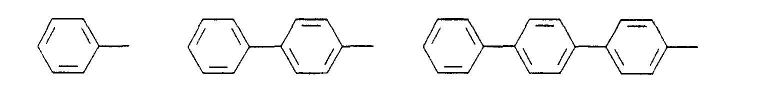 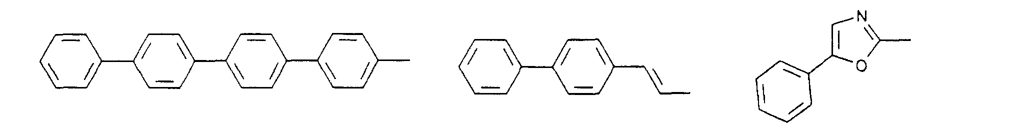 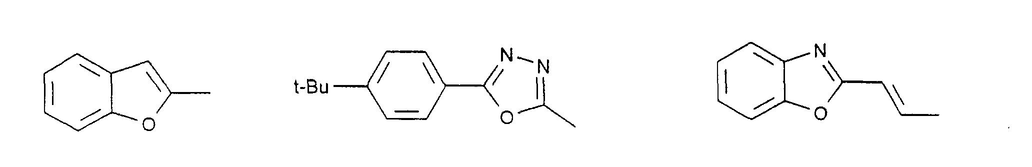 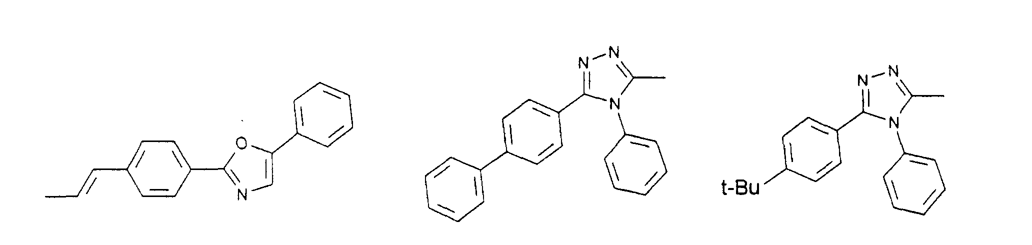 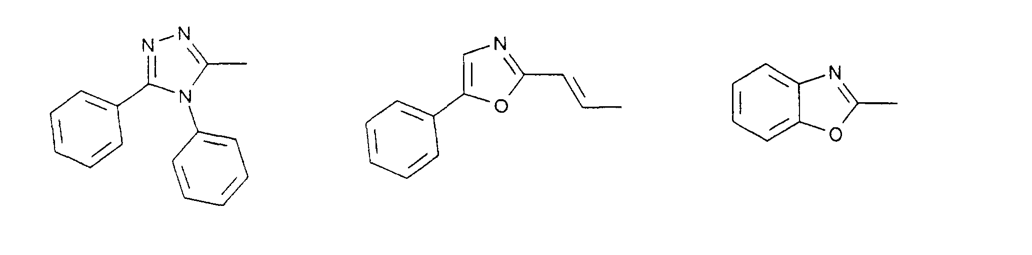 und R¹, R², R³, R⁴ sind gleich oder verschieden aus der Gruppe 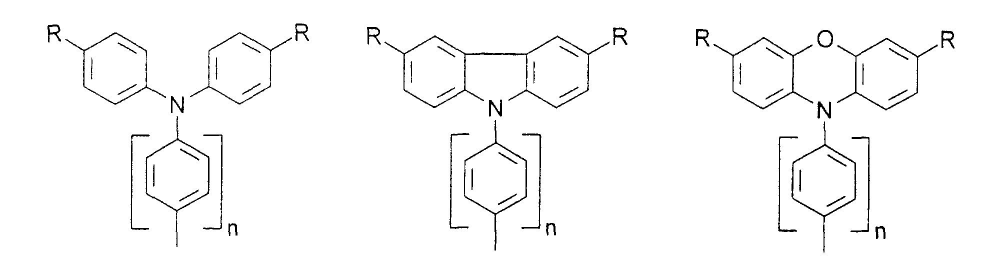 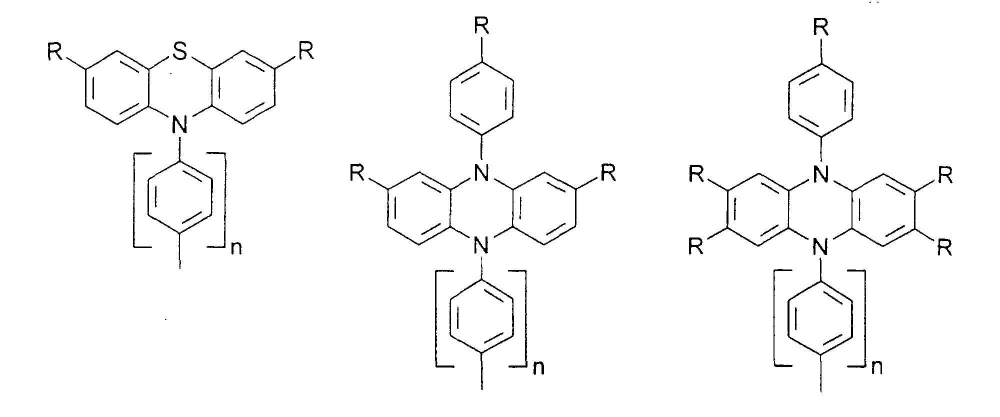 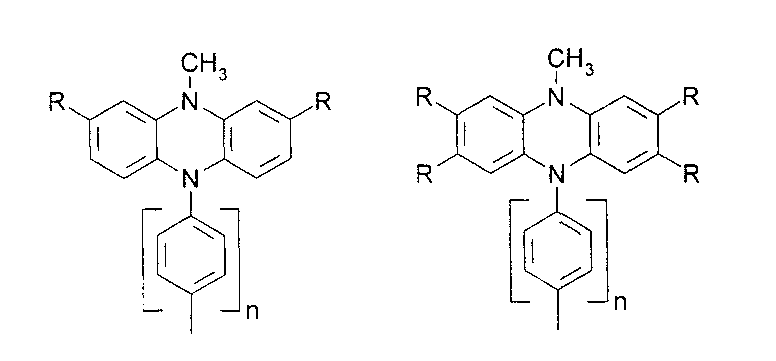 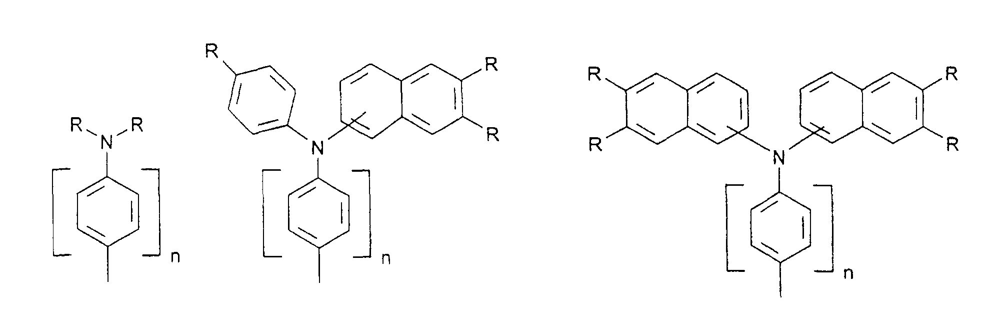 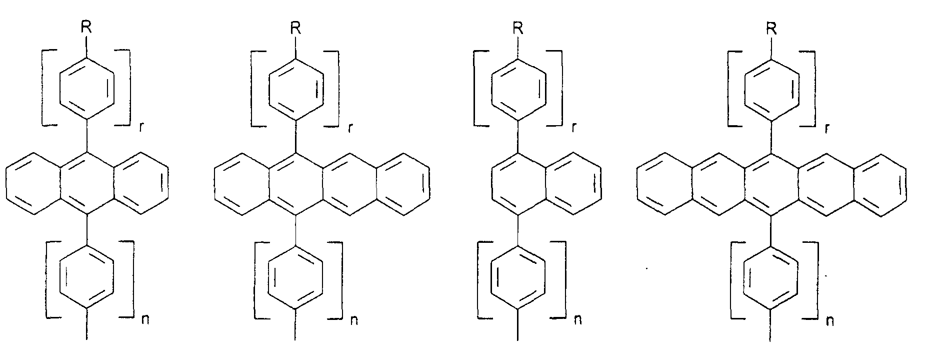 H, COOR, CH₂OR, 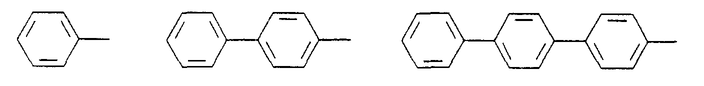 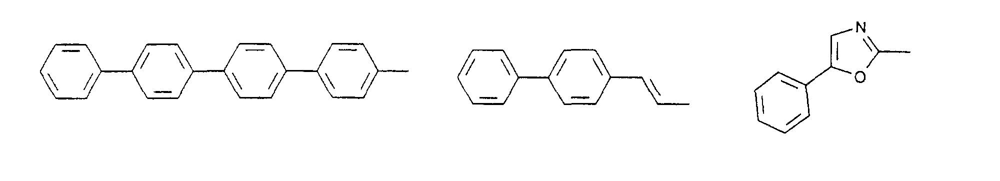 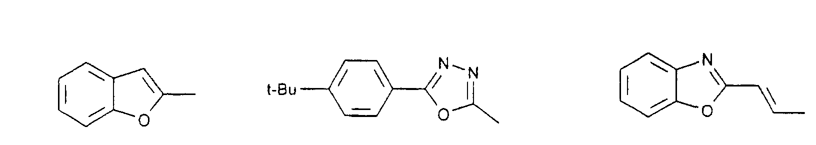 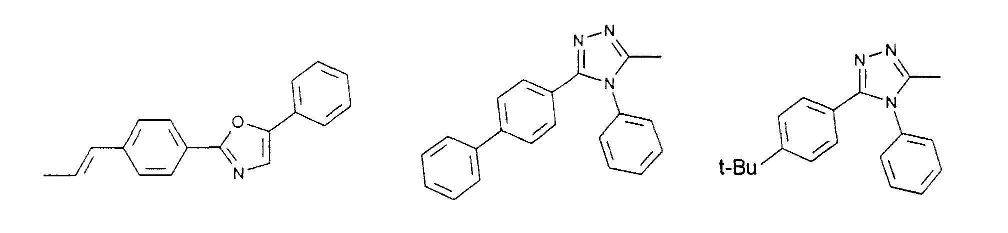 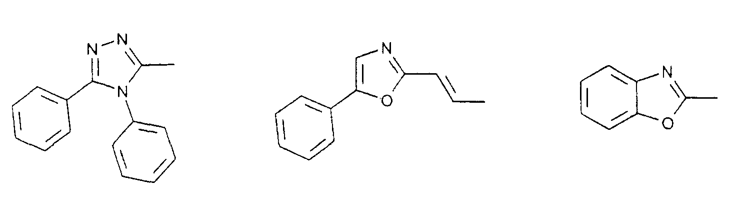 wobei die Symbole und Indizes die oben angegebenen Bedeutungen haben, und
   wobei vorzugsweise mindestens zwei der Reste R¹⁻⁴ H sind.

Insbesondere bevorzugt sind folgende Verbindungen der Formel (I):
Iaa) K¹ = L = M = N¹ und ist aus der Gruppe: 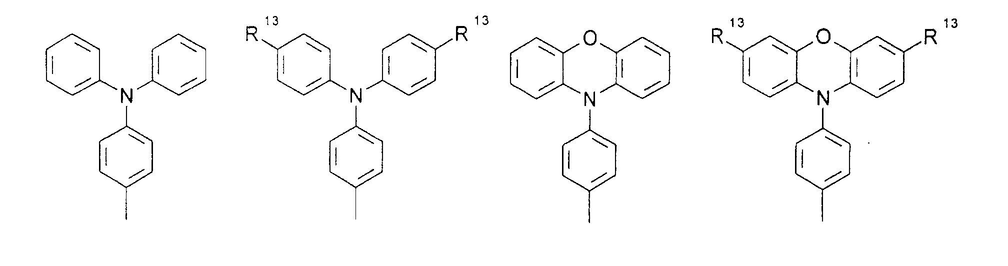 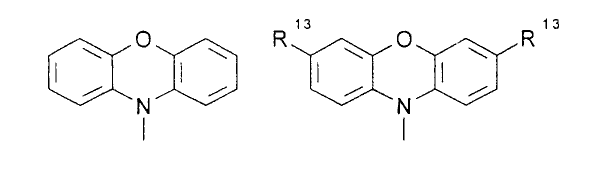 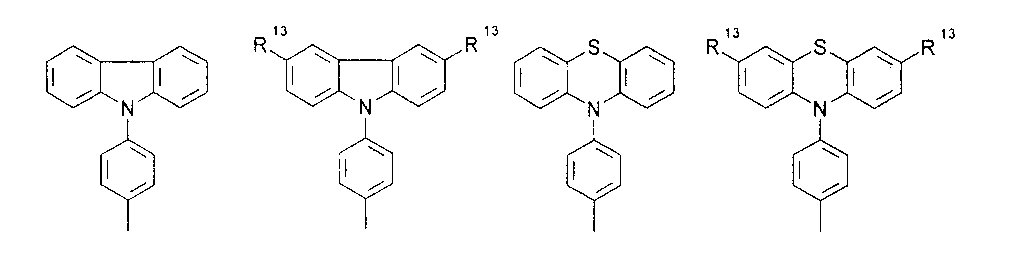 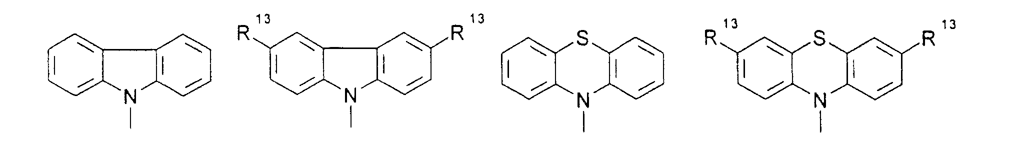 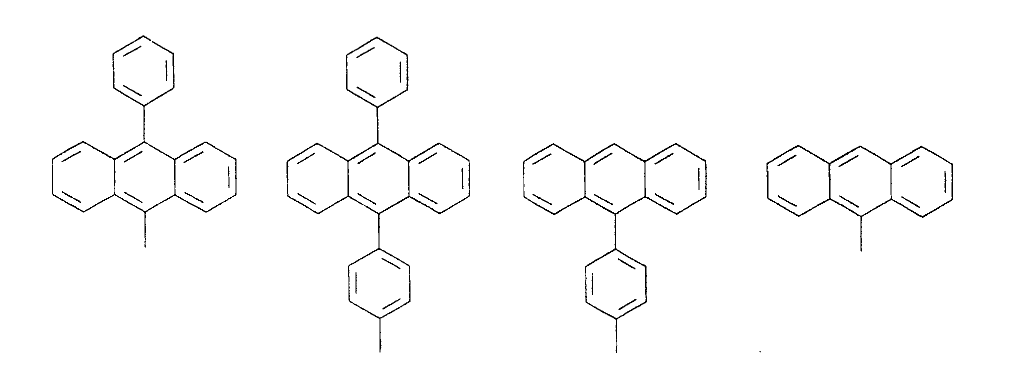 wobei R¹³ -O-CH₃, -O-C₂H₅, -S-CH₃, -S-C₂H₅, vorzugsweise -O-CH₃, -S-CH₃,
   besonders bevorzugt -O-CH₃, bedeutet;
   und R¹=R²=R³=R⁴ und ist aus der Gruppe
   H, COOR¹⁴, CH₂OR¹⁴, 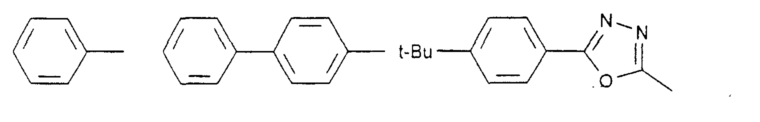 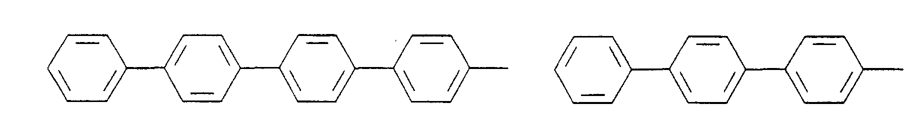 wobei R¹⁴ eine geradkettige oder verzweigte Alkylgruppe mit 1 bis 12, vorzugsweise 1 bis 4 C-Atomen ist, und wobei vorzugsweise mindestens zwei der Reste R¹⁻⁴ H sind;
I.ba) K¹ = L = M = N¹ und ist aus der Gruppe 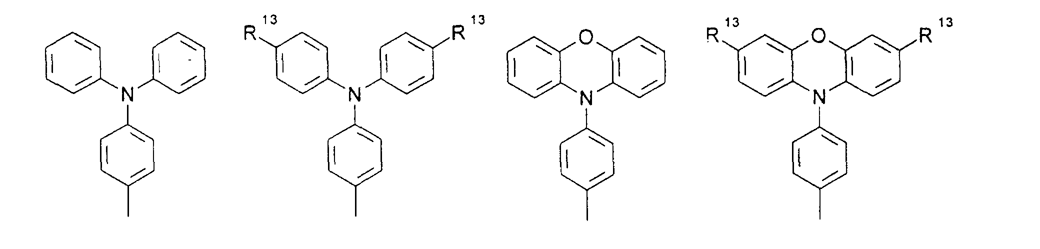 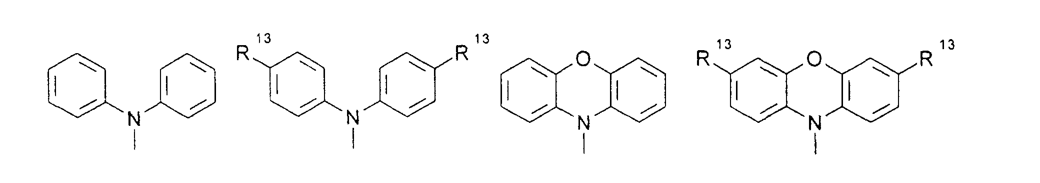 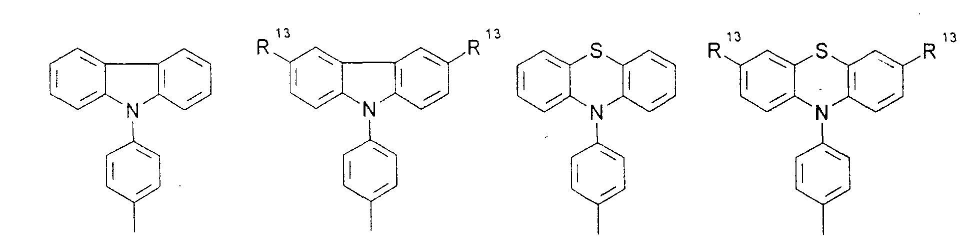 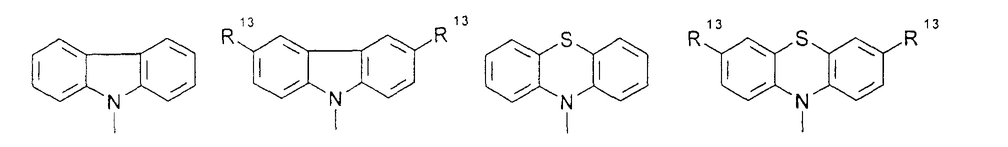 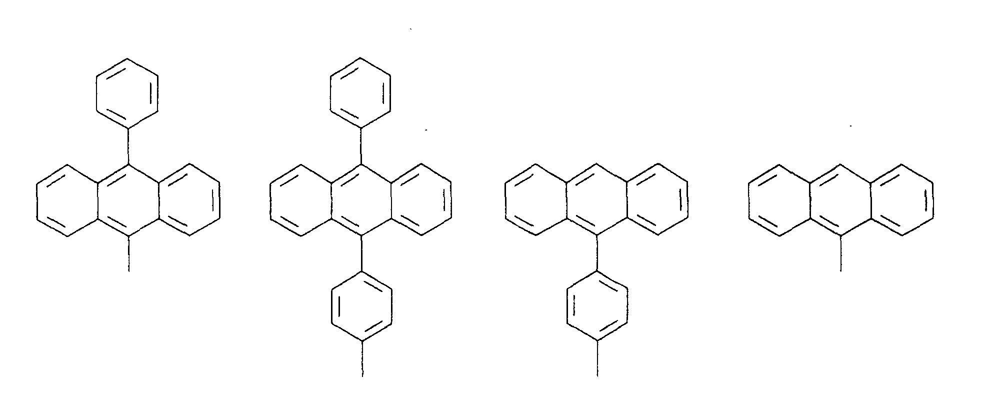 wobei R¹³ die oben angegebenen Bedeutungen hat, und R¹, R², R³, R⁴ sind H;
I.ca) K¹ = L = M = N¹ und ist aus der Gruppe 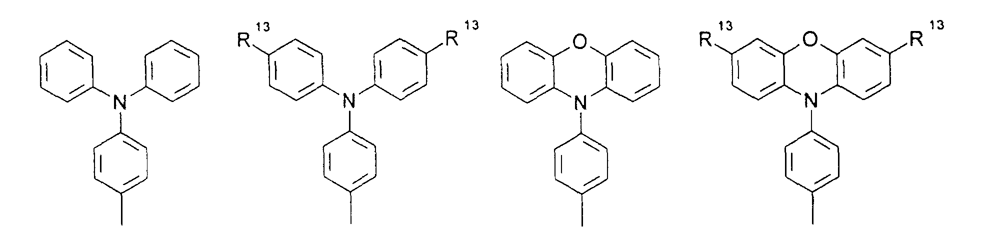 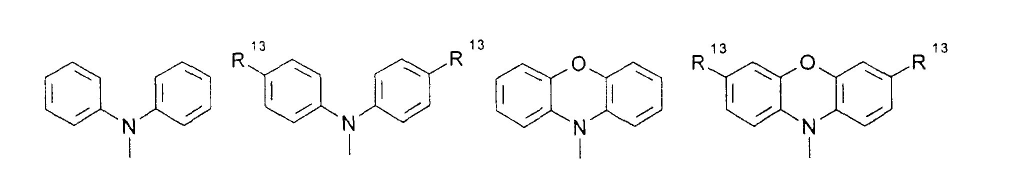 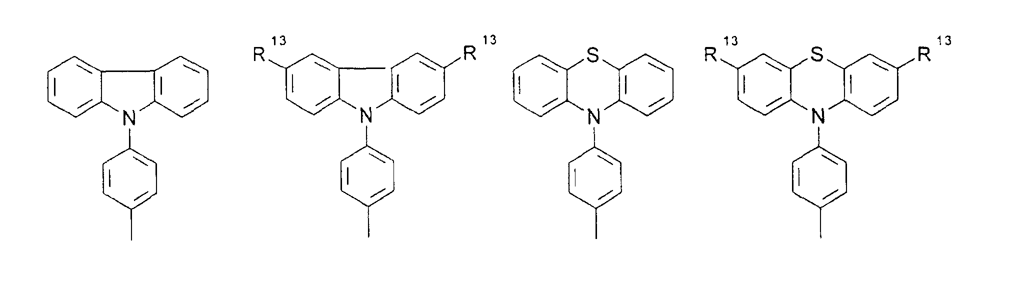 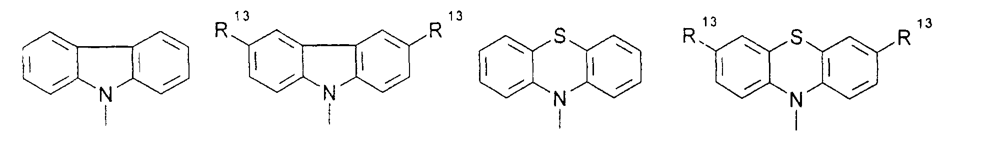 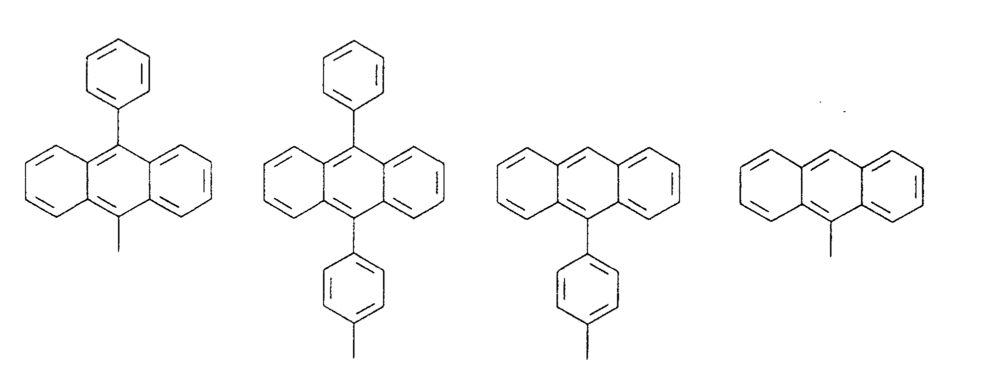 und drei der Reste R¹, R², R³, R⁴ sind H und einer ist aus der Gruppe
   H, COOR¹⁴, CH₂OR¹⁴, 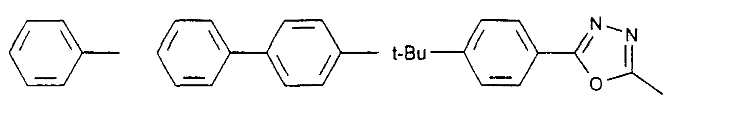 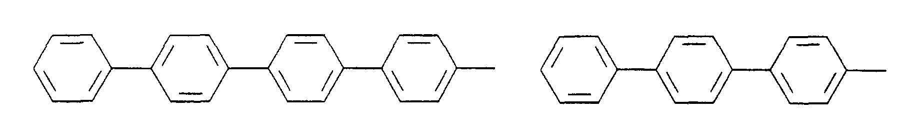 wobei R¹³, R¹⁴ die oben angegebenen Bedeutungen haben.

Die Herstellung der erfindungsgemäßen Spiroverbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart und in den entsprechenden Bänden der Serie "The Chemistry of Heterocyclic Compounds" von A. Weissberger und E.C. Taylor (Herausgeber) beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Die Herstellung von Verbindungen der Formel (la) kann beispielsweise ausgehend von einer Tetrahalogenierung des Spirogrundkörpers und anschließender Substitutionsreaktion erfolgen (analog z.B. US 5,026,894) oder über eine Tetraacetylierung mit anschließender C-C-Verknüpfung nach Umwandlung der Acetylgruppen in Aldehydgruppen oder Heterocyclenaufbau nach Umwandlung der Acetylgruppen in Carbonsäuregruppen erfolgen.

Die Herstellung von Verbindungen der Formel (lb) kann beispielsweise analog zu denen der Formel (la) erfolgen, wobei die stöchiometrischen Verhältnisse bei der Umsetzung so gewählt werden, daß die den Positionen 2,2' bzw. 7,7' des Spirofluorens entsprechenden Positionen funktionalisiert werden (siehe z.B. J. H. Weisburger, E. K. Weisburger, F. E. Ray, J. Am. Chem. Soc. 1959, 72 4253; F. K. Sutcliffe, H. M. Shahidi, D. Paterson, J. Soc. Dyers Colour 1978, 94, 306 und G. Haas, V. Prelog, Helv. Chim. Acta 1969, 52 1202).

Die Herstellung von Verbindungen der Formel (Ic) kann beispielsweise über eine Dibromierung in Stellung und anschließende Diacetylierung in mit anschließender Umsetzung analog zu den Verbindungen (la) erfolgen.

Verbindungen der Formeln (I) mit K¹, L, R¹, R², R³, R⁴ = H und M = N¹ oder R¹, R², R³, R⁴ = H, K¹ = L und M = N¹ sind beispielsweise durch Wahl geeignet substituierter Ausgangsverbindungen beim Aufbau des Spirogerüsts herstellbar.

Für die Synthese der Gruppen K¹, L, M, N¹, R¹, R², R³, R⁴ sei beispielsweise verwiesen auf DE-A 23 44 732, 24 50 088, 24 29 093, 25 02 904, 26 36 684, 27 01 591 und 27 52 975 für Verbindungen mit 1,4-Phenylen-Gruppen; DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; N. Miyaura, T. Yanagi und A. Suzuki in Synthetic Communications 1981, 11, 513 bis 519, DE-A-39 30 663; M. J. Sharp, W. Cheng, V. Snieckus, Tetrahedron Letters 1987, 28 , 5093; G. W. Gray, J. Chem. Soc. Perkin Trans II 1989, 2041 und Mol. Cryst. Liq. Cryst. 1989, 172, 165; Mol. Cryst. Liq. Cryst. 1991, 204, 43 und 91; EP-A 0 449 015; WO 89/12039; WO 89/03821; EP-A 0 354 434 für die direkte Verknüpfung von Aromaten und Heteroaromaten.

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

Aminogruppen enthaltende Verbindungen der Formel (I) können über Varianten der Ullmann-Reaktion (J. March, Adv. Org. Chem., 4. Aufl., S. 665, John Wiley & Sons, New York 1992) aufgebaut werden, wie sie bsp. In Chem. Lett. 1989, 1145; Mol. Cryst. Liq. Cryst. 1994, 242, 127 und insbesondere bei J. Salbeck et al., 213th ACS National Meeting, San Francisco 1997, Book of Abstracts, S. 199, beschrieben ist.

Weiterhin möglich ist ein aus US 5,576,460 bekanntes Verfahren. Bevorzugt ist die Herstellung solcher Verbindungen nach einem in der deutschen Patentanmeldung 197 38 860.4 mit dem Titel "Verfahren zur Hestellung von Acyloligoaminen" offenbarten Verfahren. Auf diese Anmeldung wird ausdrücklich Bezug genommen, sie gilt durch Zitat als Bestandteil der Beschreibung.

Die erfindungsgemäßen Spiroverbindungen der Formel (I) eignen sich als Ladungstransportmaterialien, vorzugsweise für photovoltaische Zellen.

Gegenstand der Erfindung ist daher auch die Verwendung von Spiroverbindungen der Formel (I) als Ladungstransportmaterial, insbesondere für photovoltaische Zellen.

Weiterhin Gegenstand der Erfindung ist eine photovoltaische Zelle mit einer Ladungstransportschicht, die eine oder mehrere, vorzugsweise eine, Spiroverbindungen der Formel (I) enthält, vorzugsweise daraus besteht.

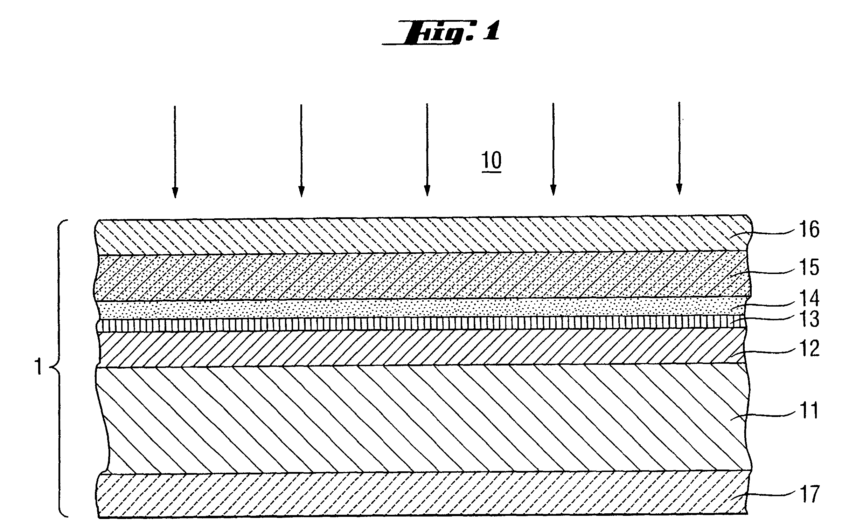

In Fig. 1 ist eine bevorzugte Ausführungsform einer solchen Zelle 1 dargestellt (nicht maßstabsgetreu).
Auf einem leitenden Träger 11, der als Elektrode bzw. Kontakt dienen kann und beispielsweise aus einem Metall oder Indium-Zinn-Oxid (ITO) besteht, ist als lichtabsorbierende Schicht ein Halbleiter 12 aufgebracht, der vorzugsweise eine Oberfläche mit einem Rauheitsfaktor > 1 hat. Vorzugsweise enthält die erfindungsgemäße Zelle auf der Oberfläche des Halbleiters einen Chromophor, hier als Chromophorschicht 13 dargestellt. Die Bezeichnung lichtabsorbierende Schicht umfaßt im Sinne der Erfindung sowohl eine Halbleiterschicht als auch eine Kombination Halbleiter/Chromophor, auch wenn die eigentliche Absorption in diesem Fall nahezu ausschließlich durch den Chromophor erfolgt. Daran schließt sich die Ladungstransportschicht 14 an, die erfindungsgemäß eine Spiroverbindung der Formel (I) enthält. Sie wird auf der einen Seite durch die Gegenelektrode 15 begrenzt, die beispielsweise aus einem leitfähigen Glas, leitfähig beschichtetem Kunststoff, aus Metall oder einem anderen leitfähigen, vorzugsweise lichtdurchlässigen Material, bestehen kann. Die Zelle 1 ist vorzugsweise oben und unten durch je eine isolierende Schicht 16 bzw. 17 abgeschlossen. Sie kann einen, in der Figur nicht gezeigten seitlichen Abschluß enthalten, beispielsweise einen Rahmen aus einem elektrisch isolierendem Material, wie Kunststoff oder Glas. Durch die Verwendung eines Lochleitermaterials anstelle des flüssigen Elektrolyten ist solch ein seitlicher Rahmen aber nicht grundsätzlich notwendig. Zumindest eine Seite der Zelle muß für das in elektrische Energie zu wandelnde Licht durchlässig sein, so daß dieses zu dem Chromophor bzw. dem Halbleiter gelangen kann.
Die Zelle enthält darüber hinaus in der Figur nicht gezeigte Vorrichtungen zur Abnahme des von der Zelle erzeugten elektrischen Stroms.

Die erfindungsgemäße photovoltarische Zelle enthält als lichtabsorbierende Schicht vorzugsweise einen Halbleiter, der vorzugsweise eine sehr große Bandlücke, besonders bevorzugt mindestens 3,0 eV, ganz besonders bevorzugt über 3,0 eV, aufweist.
Damit eignen sich vorzugsweise Metalloxid-Halbleiter, insbesondere die Oxyde der Übergangsmetalle, sowie der Elemente der dritten Hauptgruppe und der vierten, fünften und sechsten Nebengruppe (des periodischen Systems der Elemente) von Titan, Zirkon, Hafnium, Strontium, Zink, Indium, Yttrium, Lanthan, Vanadium, Niob, Tantal, Chrom, Molybdän, Wolfram, aber auch Oxide von Zink, Eisen, Nickel oder Silber, Perovskite wie SrTiO₃, CaTiO₃, oder Oxide von anderen Metallen der zweiten und dritten Hauptgruppe oder Mischoxide oder Oxidgemische dieser Metalle. Es kann aber auch jedes andere Metalloxid mit Halbleitereigenschaften und großem Energieabstand (Bandlücke) zwischen Valenzband und Leitungband verwendet werden.
Besonders bevorzugt als Halbleitermaterial ist Titandioxid.

Der Halbleiter weist vorzugsweise einen Rauheitsfaktor von größer als 1, besonders bevorzugt von größer als 20, ganz besonders von größer als 150 auf.
Der Rauheitsfaktor ist definiert als das Verhältnis einer wirklichen/effektiven Oberfläche zur Fläche der Projektion dieser Oberfläche eines Körpers, in diesem Fall also der Oberfläche des Halbleiters.

Der Rauheitsfaktor kann z.B.durch gravimetrische Adsorptionsmethoden bestimmt werden, wie z.B. in F. Kohlrausch, Praktische Physik, Band 1,
S. 397 (Stuttgart: B.G. Teubner, 1985) beschrieben wird. Im allgemeinen beträgt die Größe der Poren 5-200 nm, vorzugsweise 10-50 nm.

Ein Verfahren zum Herstellen von polykristallinen Metalloxid-Halbleiterschichten mit dem Sol-Gel-Verfahren (beschrieben in Einzelheiten z. B. in Stalder und Augustynski, J. Electrochem. Soc. 1979, 126, 2007), wo beim Verfahrensschritt der Hydrolyse des Metall-Alkoholats die prozentuale relative Feuchtigkeit der Umgebungsatmosphäre in einem Bereich von 30 % bis 80 % liegen kann und innerhalb von ± 5 %, vorzugsweise ± 1 %, konstant gehalten wird, ergibt Metalloxid-Halbleiterschichten, mit denen in erfindungsgemäßen photovoltaischen Zellen eine besonders hohe elektrische Ausbeute erzielt werden kann.
Die rauhe Oberfläche mit polykristalliner Struktur bietet eine um den Rauheitsfaktor größere Fläche für eine, vorzugsweise, monomolekulare Oberflächenschicht eines Chromophors. Damit wird erreicht, daß das auf eine Fläche bestimmter Größe einfallende Licht mit bedeutend höherer Ausbeute in elektrische Energie gewandelt wird. Der Halbleiter kann als für den einfallenden Lichtstrom durchsichtig betrachtet werden. Licht wird aber teilweise auf der Oberfläche reflektiert und gelangt z. T. auf benachbarte Oberflächen. Das in den Halbleiter eindringende und nicht absorbierte bzw. gewandelte Licht trifft z. T. direkt und zu einem anderen Teil indirekt und zu einem weiteren Teil indirekt nach Totalreflexion an der Oberfläche, auf der Austrittsseite auf Chromophormoleküle, womit es gelingt, eine bedeutend höhere Lichtausbeute zu erzielen.

Die Empfindlichkeit, d. h. die photoelektronische Ausbeute für sichtbares Licht, also auch für Sonnenlicht, kann also erhöht werden, indem auf der Oberfläche des Halbleiters sog. Chromophore, auch Sensibilatoren oder Dyes genannt, als Ladungsträger chemisch an- oder eingelagert (chemisorbiert) werden. Die beiden Funktionen der Lichtabsorption und der Ladungsträger-Trennung sind bei diesen photoelektronischen Systemen getrennt. Die Lichtabsorption wird vom Chromophor im Oberflächenbereich übernommen, und die Trennung der Ladungsträger erfolgt an der Grenzschicht Halbleiter/Chromophor.
Verschiedene Chromophore haben unterschiedliche spektrale Empfindlichkeiten. Die Wahl des Chromophors kann somit der spektralen Zusammensetzung des Lichts der Lichtquelle angepaßt werden, um die Ausbeute möglichst zu vergrößern. Als Chromophore, d. h. Sensibilisatoren, eigenen sich insbesondere die Komplexe von Übergangsmetallen vom Typ Metall (L₃), Metall (L₂) von Ruthenium und Osmium (z. B. Ruthenium-tris-(2,2'-bipyridyl-4,4'-dicarbonsäure) und deren Salze, Ruthenium cis diaqua bipyridyl Komplexe, wie Ruthenium cis-diaqua bis(2,2'bipyridyl-4,4'dicarboxylate) sowie Porphyrine (z. B. Zink tetra (4-carboxyphenyl) Porphyrin) und Cyanide (z. B. Eisen-Hexacyanid-Komplexe) und Phthalocyanine.
Die Chromophore können im Bereich der Oberfläche des Metalloxyd-Halbleiters chemisorbiert, adsorbiert oder sonstwie fest angelagert sein. Günstige Resultate wurden beispielsweise mit Chromophoren erzielt, die mit Carbonsäure- oder Phosphonsäure-Liganden an die Oberfläche des Metalloxyd-Halbleiters gebunden sind.

Geeignete Chromophore sind beispielsweise in Chem. Rev. 1995, 49-68 beschrieben.

Besonders bevorzugt sind Ruthenium-tris-(2,2'-bipyridyl-4,4'-dicarbonsäure), deren Salze und die Chromophore (VIII) und (IX), 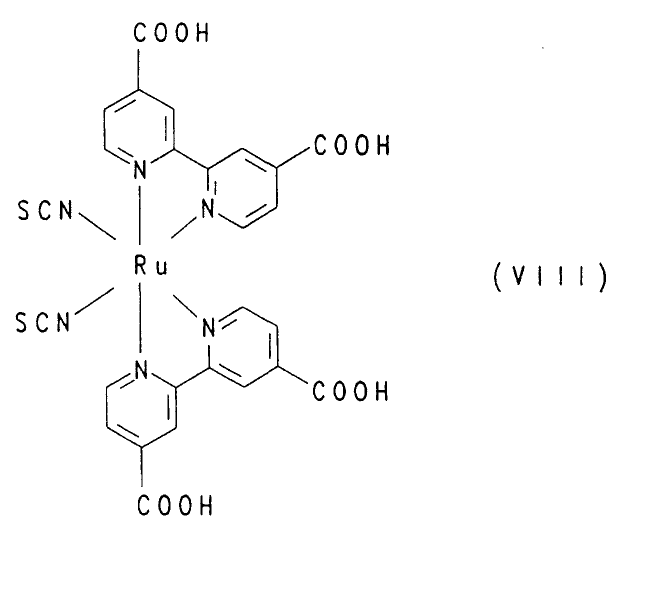 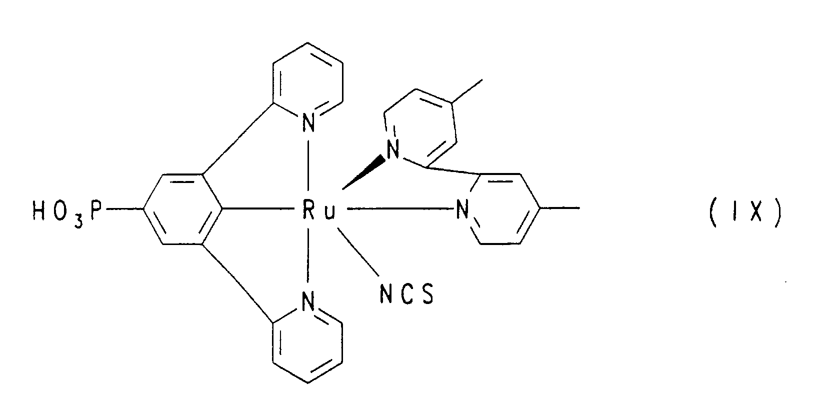 deren Synthese und Eigenschaften in J. Chem. Soc. Chem. Comm. 1995, 65 beschrieben sind.

Das Aufbringen des Chromophors, beispielsweise Ruthenium-tris-(2,2'-bipyridyl-4,4'-dicarbonsäure) oder ein Salz davon, erfolgt z. B. durch Eintauchen des Substrats mit der Oxidschicht in eine Lösung, beispielsweise eine wäßrige oder alkoholische, vorzugsweise ethanolische, während etwa einer Stunde. Die Konzentration der Lösung ist vorzugsweise 0,1 bis 10 molar, besonders bevorzugt 1 bis 5 molar, beispielsweise 2 molar. Andere Chromophore lassen sich nach analogen Verfahren auf Titanoxid oder andere Metalloxid-Halbleiter aufbringen.

Als Elektrode 15 eignen sich stabile, metallisch leitende Substanzen, z. B. Au, Ag, Pt, Cu, oder andere Metalle. Es können aber auch, für einige Anwendungen vorzugsweise lichtdurchlässige leitfähige Substanzen, wie dotierte Metalloxide, z. B. Indium-Zinn-Oxid, Sb-dotiertes Zinnoxid oder Al-dotiertes Zinkoxid, verwendet werden. Dabei kann die Austrittsarbeit des verwendeten Elektrodenmaterials vorzugsweise an das lonisationspotential des verwendeten Lochtransportmaterials angepaßt werden.

Die Elektrode kann, wie in der EP-A 0 333 641 beschrieben, auf ein transparentes Substrat, z. B. Glas, aufgebracht werden und mit der Lochtransportschicht verbunden sein. Vorzugsweise kann sie in der erfindungsgemäßen Zelle durch physikalische Abscheidemethoden, z. B. Aufdampfen oder Zerstäuben (Sputtern), direkt auf die Lochtransportschicht aufgebracht werden, ohne daß eine zweite Glasplatte notwendig ist. Dieses Verfahren ist in Anwendungen zu bevorzugen, bei denen das Gewicht der Zelle vermindert werden soll.

Falls gewünscht kann die Zelle z. B. mit einem Kleber oder einer Folie versiegelt werden.

Eine erfindungsgemäße photovoltaische Zelle hat im allgemeinen eine Dicke von 5 bis 20 mm (mit Substrat).
Sie kann zur Vermeidung von Reflexionsverlusten mit einer ein-, zwei- oder mehrlagigen Antireflexschicht versehen sein.

Spiroverbindungen der Formel (I) eignen sich weiterhin als Elektrolumineszenzmaterialien.

Als Elektrolumineszenzmaterialien im Sinne der Erfindung gelten Materialien, die als aktive Schicht in einer Elektrolumineszenzvorrichtung Verwendung finden können. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung ist daher auch die Verwendung einer Spiroverbindung der Formel (I) als Elektrolumineszenzmaterial, d.h. als aktive Schicht in einer Elektrolumineszensvorrichtung.

Um als Elektrolumineszenzmaterialien Verwendung zu finden, werden die Spiroverbindungen der Formel (I) im allgemeinen nach bekannten, dem Fachmann geläufigen Methoden, wie Eintauchen (Dipping) oder Spincoating, in Form eines Films auf ein Substrat aufgebracht.

Gegenstand der Erfindung ist weiterhin eine Elektrolumineszenzvorrichtung mit einer oder mehreren aktiven Schichten, wobei mindestens eine dieser aktiven Schichten ein oder mehrere erfindungsgemäße Spiroverbindungen der Formel (I) enthält. Die aktive Schicht kann beispielsweise eine lichtemittierende Schicht und/oder eine Transportschicht und/oder eine Ladungsinjektionsschicht sein.

Der allgemeine Aufbau solcher Elektrolumineszenzvorrichtungen ist beispielsweise in US 4,539,507 und US 5,151,629 beschrieben. Polymere enthaltende Elektrolumineszenzvorrichtungen sind beispielsweise in WO-A 90/13148 oder EP-A 0 443 861 beschrieben.

Sie enthalten üblicherweise eine elektrolumineszierende Schicht zwischen einer Kathode und einer Anode, wobei mindestens eine der Elektroden transparent ist. Zusätzlich können zwischen der elektrolumineszierenden Schicht und der Kathode eine oder mehrere Elektroneninjektions- und/oder Elektronentransportschichten eingebracht sein und/oder zwischen der elektrolumineszierenden Schicht und der Anode eine oder mehrere Lochinjektions- und/oder Lochtransportschichten eingebracht sein. Als Kathode dienen vorzugsweise Metalle oder metallische Legierungen, z.B. Ca, Mg, Al, In, Mg/Ag. Als Anode dienen Metalle, z.B. Au, oder andere metallisch leitende Stoffe, wie Oxide, z.B. ITO (Indiumoxid/Zinnoxid) auf einem transparentem Substrat, z.B. aus Glas oder einem transparenten Polymer.

Im Betrieb wird die Kathode auf negatives Potential gegenüber der Anode gesetzt. Dabei werden Elektronen von der Kathode in die Elektroneninjektionsschicht/Elektronentransportschicht oder direkt in die lichtemittierende Schicht injiziert. Gleichzeitig werden Löcher von der Anode in die Lochinjektionsschicht/Lochtransportschicht oder direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht oder innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendeten Materialien variiert werden.

Elektrolumineszenzvorrichtungen finden z.B. Anwendung als selbstleuchtende Anzeigeelemente, wie Kontrollampen, alphanumerische Displays, Hinweisschilder, und in optoelektronischen Kopplern.

## Patentansprüche

1. Spiroverbindungen der allgemeinen Formel (I), 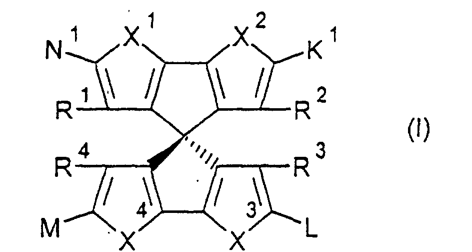 wobei die Symbole folgende Bedeutungen haben:
X¹, X², X³, X⁴ sind gleich oder verschieden -S-, -O-, -NR⁵-, -CR⁵=N-, -CR⁵=CH-, mit der Maßgabe, daß mindestens eine der Gruppen X¹⁻⁴ von -CR⁵=CH- verschieden ist;
K¹, L, M, N¹, R¹, R², R³, R⁴ sind gleich oder verschieden und bedeuten
a) Wasserstoff, -NO₂, -CN, -F oder -Cl,
b) ein geradkettiger oder verzweigter Alkylrest mit 1 bis 20 C-Atomen, wobei
b1 ) eine oder mehrere nicht benachbarte CH₂-Gruppen durch -O-, -S-, -CO-O-, -O-CO-, -O-CO-O-, NR⁵ oder -Si(CH₃)₂- ersetzt sein können und/oder
b2) eine oder mehrere CH₂-Gruppen durch -CH=CH-, -C≡C-, 1,4-Phenylen, 1,4-Cyclohexylen oder 1,3-Cyclopentylen ersetzt sein können und/oder
b3) ein oder mehrere H-Atome durch F und/oder Cl ersetzt sein können und/oder
c) eine der folgenden Gruppen: 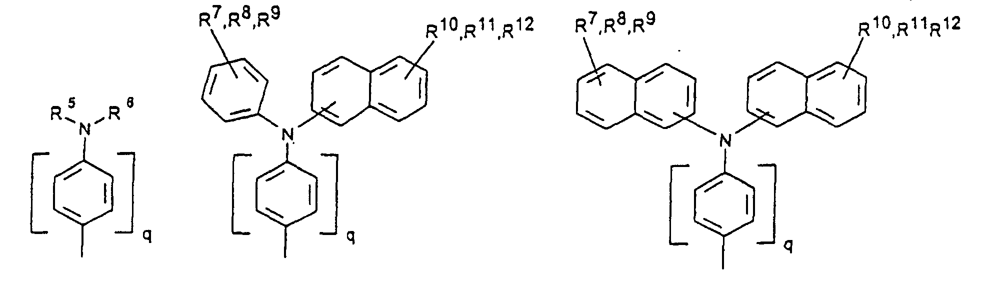 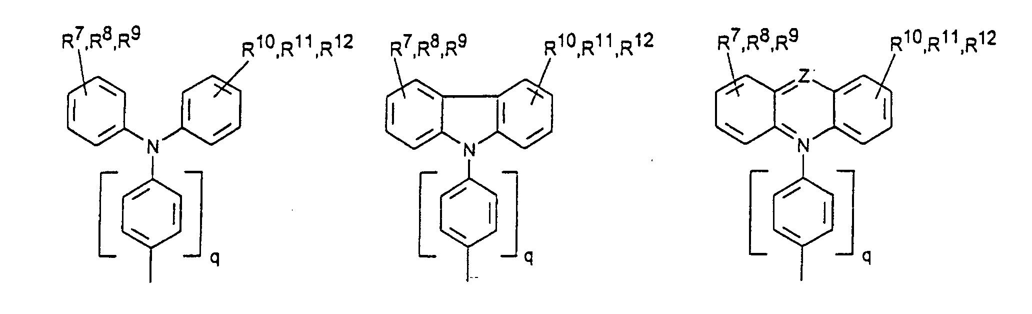 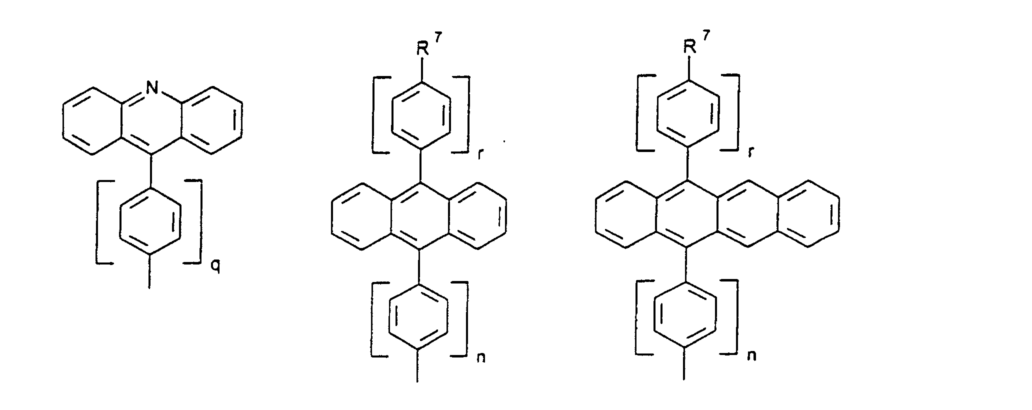 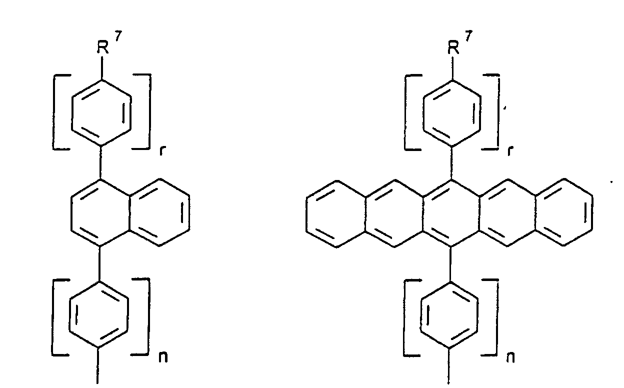 oder 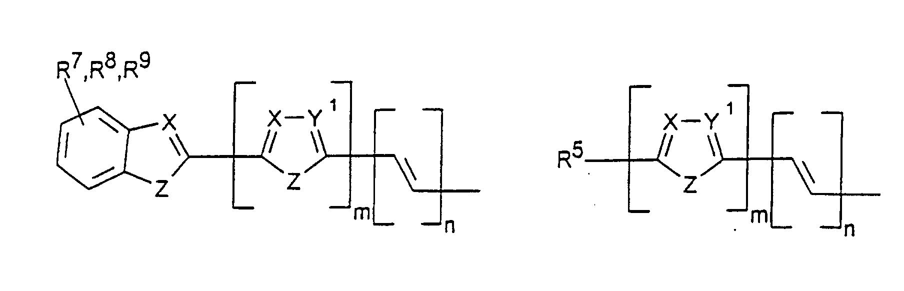 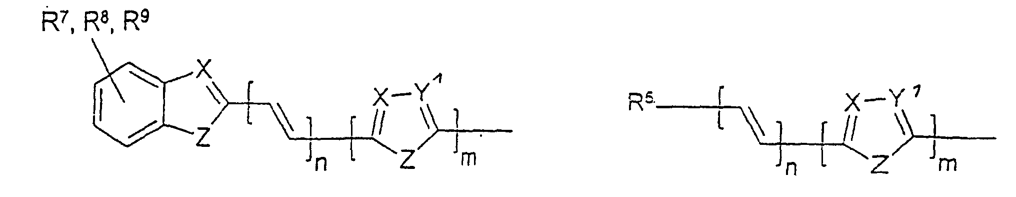
X, Y¹ sind jeweils gleich oder verschieden =CR⁷- oder =N-;
Z ist -O-, -S-, =NR⁵-,
R⁵, R⁶ sind jeweils gleich oder verschieden
a) Wasserstoff
b) ein geradkettiger oder verzweigter Alkylrest mit 1 bis 20 C-Atomen, wobei
b1 ) eine oder mehrere nicht benachbarte und nicht an Stickstoff gebundene CH₂-Gruppen durch -O-, -S-, -CO-O-, -O-CO-, -O-CO-O- oder -Si(CH₃)₂ ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F und/oder Cl ersetzt sein können und/oder
b4) R⁵ und R⁶ zusammen auch einen Ring bilden können;
c) Phenyl, Biphenyl, 1-Naphthyl, 2-Naphthyl, 2-Thienyl, 2-Furanyl;
R⁷, R⁸, R⁹, R¹⁰, R¹¹, R¹² sind gleich oder verschieden
a) Wasserstoff, -CN, -F, NO₂ oder -Cl
b) ein geradkettiger oder verzweigter Alkylrest mit 1 bis 20 C-Atomen,
wobei
b1 ) eine oder mehrere nicht benachbarte CH₂-Gruppen durch -O-, -S-, -CO-O-, -O-CO-, -O-CO-O-, -NR⁵ oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F und/oder Cl ersetzt sein können;
c) Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl,
m, n, p, q, r sind jeweils gleich oder verschieden 0, 1, 2, 3, 4, 5 oder 6,
ausgenommen die Verbindungen der Formel 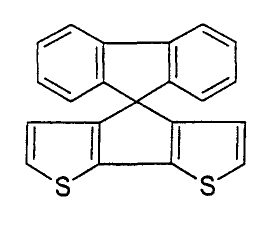 sowie 4,5-Diazospirobifluoren und 4,4',5,5'-Tetraazospirobifluoren.

2. Spiroverbindungen gemäß Formel (1) in Anspruch 1, **dadurch gekennzeichnet, daß**:
a) X¹⁻⁴ identisch sind oder
b) X¹ gleich X² und X³ gleich X⁴ ist.

3. Spiroverbindungen gemäß Anspruch 1 und/oder 2, aus der Gruppe
a) 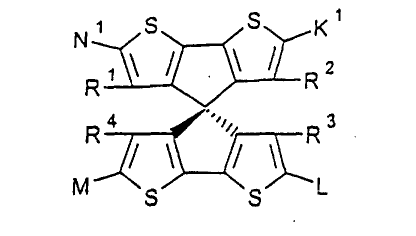
b) 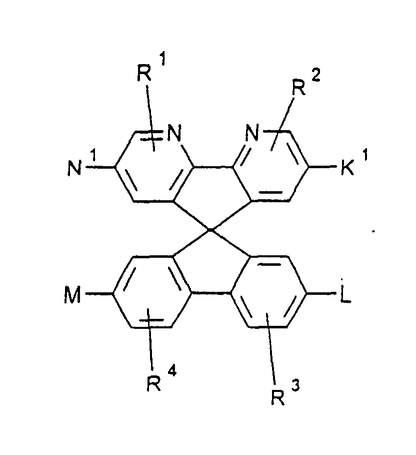
c)

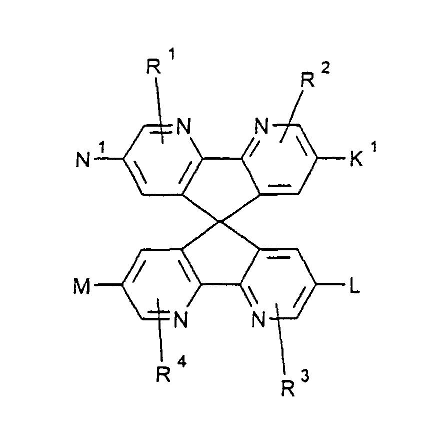

4. Verwendung einer amorphen Spiroverbindung der allgemeinen Formel (I) 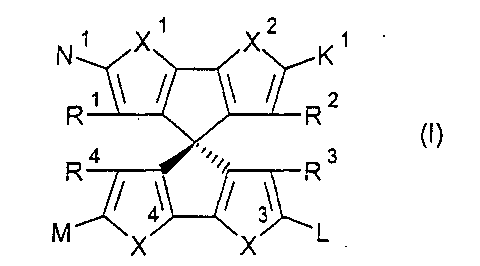 wobei die Symbole folgende Bedeutungen haben:
X¹, X², X³, X⁴ sind gleich oder verschieden -S-, -O-, -NR⁵-, -CR⁵=N-, -CR⁵=CH-, mit der Maßgabe, daß mindestens eine der Gruppen X¹⁻⁴ von -CR⁵=CH- verschieden ist;
K¹, L, M, N¹, R¹, R², R³, R⁴ sind gleich oder verschieden und bedeuten
a) Wasserstoff, -NO₂, -CN, -F oder -Cl,
b) ein geradkettiger oder verzweigter Alkylrest mit 1 bis 20 C-Atomen, wobei
b1) eine oder mehrere nicht benachbarte CH₂-Gruppen durch -O-, -S-, -CO-O-, -O-CO-, -O-CO-O-, NR⁵ oder -Si(CH₃)₂- ersetzt sein können und/oder
b2) eine oder mehrere CH₂-Gruppen durch -CH=CH-, -C≡C-, 1,4-Phenylen, 1,4-Cyclohexylen oder 1,3-Cyclopentylen ersetzt sein können und/oder
b3) ein oder mehrere H-Atome durch F und/oder Cl ersetzt sein können und/oder
c) eine der folgenden Gruppen: 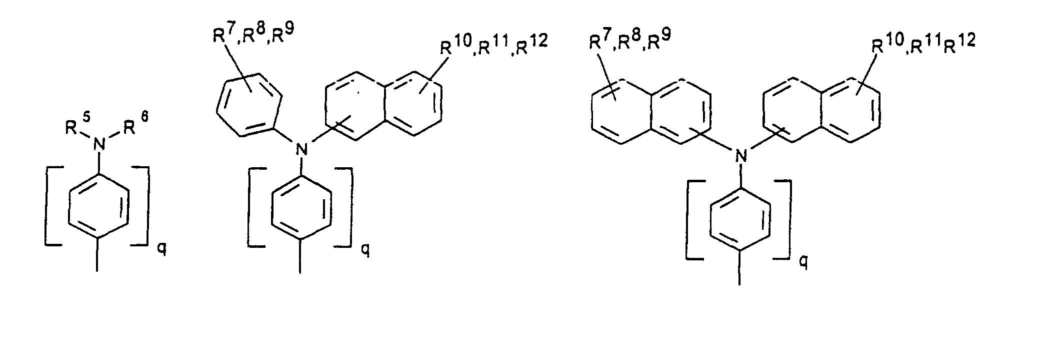 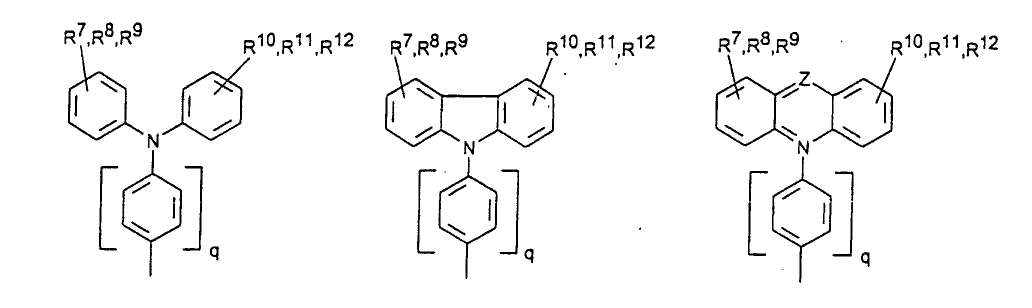 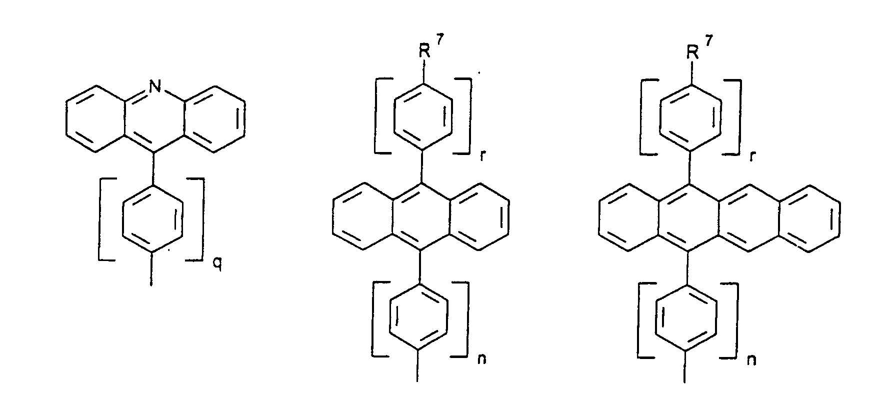 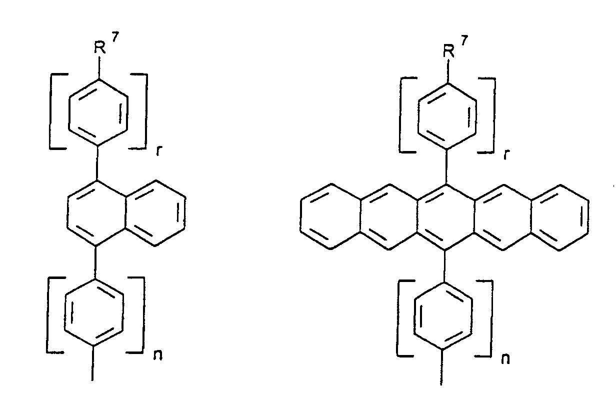 oder 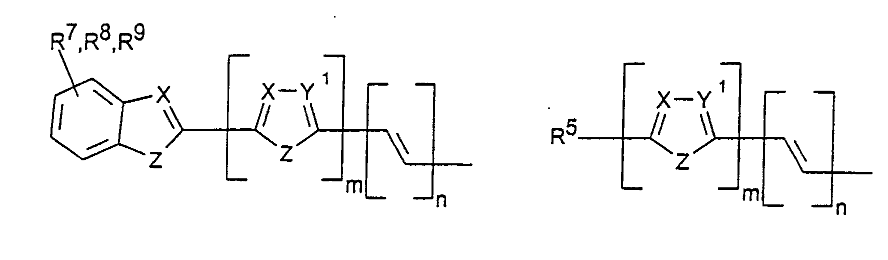 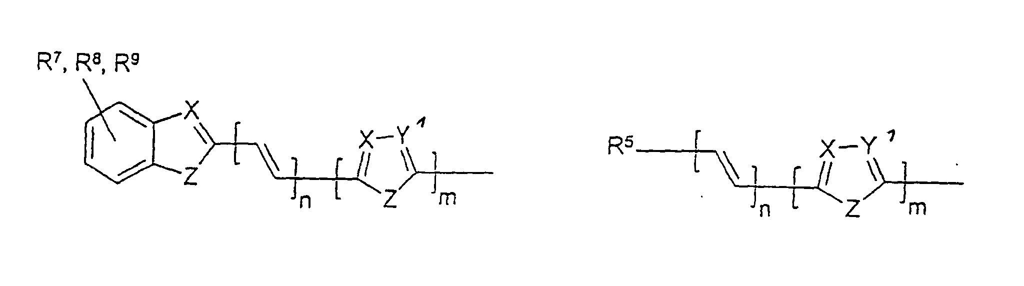
X, Y¹ sind jeweils gleich oder verschieden =CR⁷- oder =N-;
Z ist -O-, -S-, -NR⁵-,
R⁵, R⁶ sind jeweils gleich oder verschieden
a) Wasserstoff
b) ein geradkettiger oder verzweigter Alkylrest mit 1 bis 20 C-Atomen, wobei
b1 ) eine oder mehrere nicht benachbarte und nicht an Stickstoff gebundene CH₂-Gruppen durch -O-, -S-, -CO-O-, -O-CO-, -O-CO-O- oder -Si(CH₃)₂ ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F und/oder Cl ersetzt sein können und/oder
b4) R⁵ und R⁶ zusammen auch einen Ring bilden können;
c) Phenyl, Biphenyl, 1-Naphthyl, 2-Naphthyl, 2-Thienyl, 2-Furanyl;
R⁷, R⁸, R⁹, R¹⁰, R¹¹, R¹² sind gleich oder verschieden
a) Wasserstoff, -CN, -F, NO₂ oder -Cl
b) ein geradkettiger oder verzweigter Alkylrest mit 1 bis 20 C-Atomen, wobei
b1 ) eine oder mehrere nicht benachbarte CH₂-Gruppen durch -O-, -S-, -CO-O-, -O-CO-, -O-CO-O-, -NR⁵ oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F und/oder Cl ersetzt sein können;
c) Phenyl, Biphenyl, 1-Naphthyl, 2-Naphthyl, 2-Thienyl, 2-Furanyl, -O-Phenyl, -O-Biphenyl, -O-1-Naphthyl, -O-2-Naphthyl, -O-2-Thienyl, -O-2-Furanyl,
m, n, p, q, r sind jeweils gleich oder verschieden 0, 1, 2, 3, 4, 5 oder 6.
als Ladungstransportmaterial in photovoltaischen Zellen.

5. Photovoltaische Zelle, enthaltend eine Ladungstransportschicht, die eine oder mehrere Spiroverbindungen gemäß Anspruch 4 enthält.

## Claims

1. A spiro compound of the formula (I), 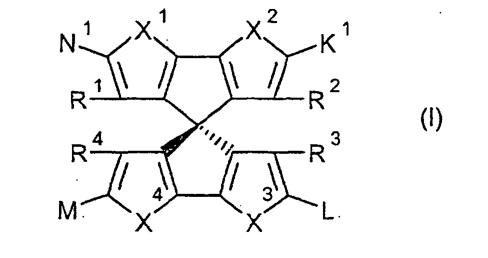 where the symbols have the following meanings:
X¹, X², X³, X⁴ are identical or different and are -S-, -O-, -NR⁵-, -CR⁵=N-, -CR⁵=CH-,
with the proviso that at least one of the groups X¹⁻⁴ is different from -CR⁵=CH-;
K¹, L, M, N¹, R¹, R², R³, R⁴ are identical or different and are each
a) hydrogen, -NO₂, -CN, -F or -Cl,
b) a straight-chain or branched alkyl radical having from 1 to 20 carbon atoms, where
b1) one or more nonadjacent CH₂ groups can be replaced by -O-, -S-, -CO-O-, -O-CO-, -O-CO-O-, NR⁵ or -Si(CH₃)₂- and/or
b2) one or more CH₂ groups can be replaced by - CH=CH-, -C≡C-, 1,4-phenylene, 1,4-cyclohexylene or 1,3-cyclopentylene and/or
b3) one or more H atoms can be replaced by F and/or Cl and/or
c) one of the following groups: 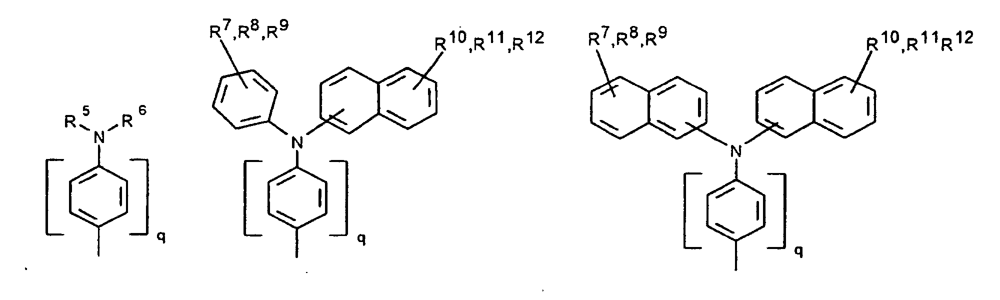 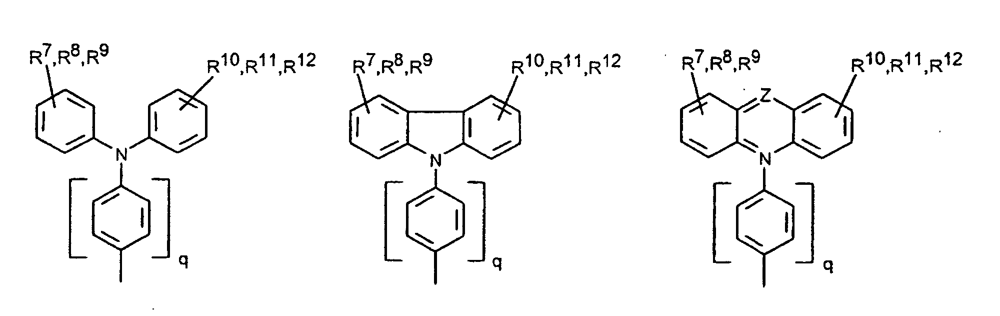 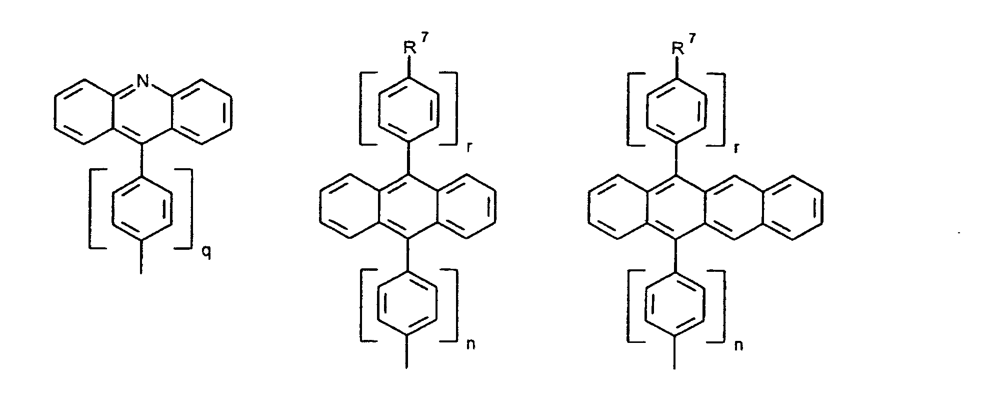 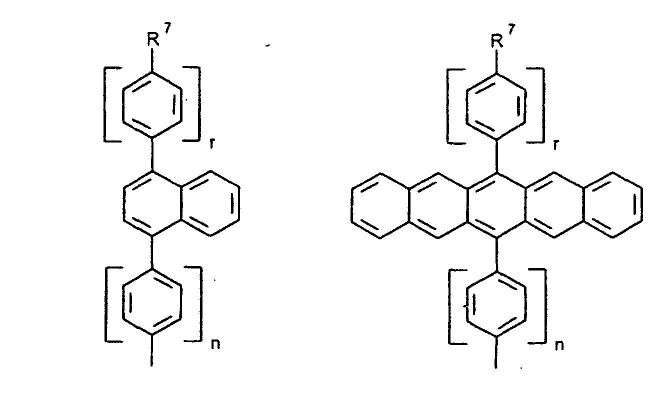 or 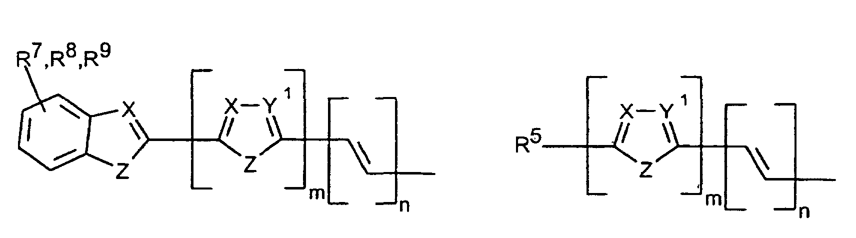
X, Y¹ are in each case identical or different and are =CR⁷- or =N-;
Z is -O-, -S-, -NR⁵-,
R⁵, R⁶ are in each case identical or different and are each
a) hydrogen
b) a straight-chain or branched alkyl radical having from 1 to 20 carbon atoms, where
b1) one or more nonadjacent CH₂ groups which are not bound to nitrogen can be replaced by -O-, -S-, -CO-O-, - O-CO-, -O-CO-O- or -Si(CH₃)₂ and/or
b2) one or more CH₂ groups can be replaced by -CH=CH-, -C≡C-, cyclopropane-1,2-diyl, 1,4-phenylene, 1,4-cyclohexylene or 1,3-cyclopentylene and/or
b3) one or more H atoms can be replaced by F and/or Cl and/or
b4) R⁵ and R⁶ together can also form a ring;
c) phenyl, biphenyl, 1-naphthyl, 2-naphthyl, 2-thienyl, 2-furanyl;
R⁷, R⁸, R⁹, R¹⁰, R¹¹, R¹² are identical or different and are each
a) hydrogen, -CN, -F, NO₂ or -Cl
b) a straight-chain or branched alkyl radical having from 1 to 20 carbon atoms, where
b1) one or more nonadjacent CH₂ groups can be replaced by -O-, -S-, -CO-O-, -O-CO-, -O-CO-O-, -NR⁵ or -Si(CH₃)₂- and/or
b2) one or more CH₂ groups can be replaced by -CH=CH-, -C≡C-, cyclopropane-1,2-diyl, 1,4-phenylene, 1,4-cyclohexylene or 1,3-cyclopentylene and/or
b3) one or more H atoms can be replaced by F and/or Cl;
c) phenyl, biphenyl, 1-naphthyl, 2-naphthyl, 2-thienyl, 2-furanyl, -O-phenyl, -O-biphenyl, -O-1-naphthyl, -O-2-naphthyl, -O-2-thienyl, -O-2-furanyl,
m, n, p, q, r are in each case identical or different and are 0, 1, 2, 3, 4, 5 or 6,
with the exception of the compounds of the formula 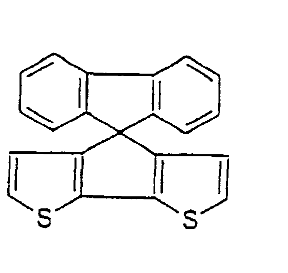 and also of 4,5-diazospirobifluorene and 4,4',5,5'-tetraazospirobifluorene.

2. A spiro compound of the formula (I) in claim 1, wherein:
a) X¹⁻⁴ are identical or
b) X¹ is identical to X² and X³ is identical to X⁴.

3. A spiro compound as claimed in claim 1 and/or 2 selected from the group consisting of
a) 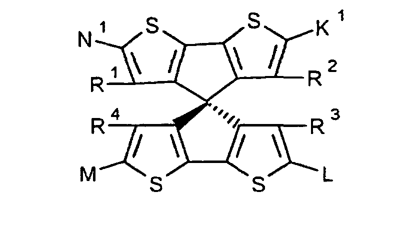
b) 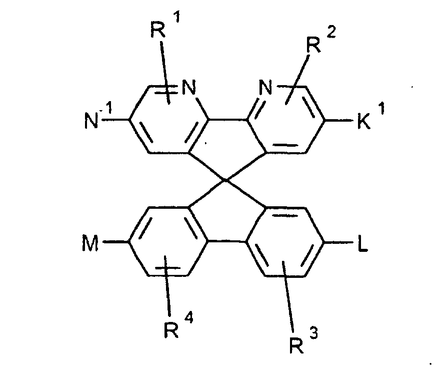
c)

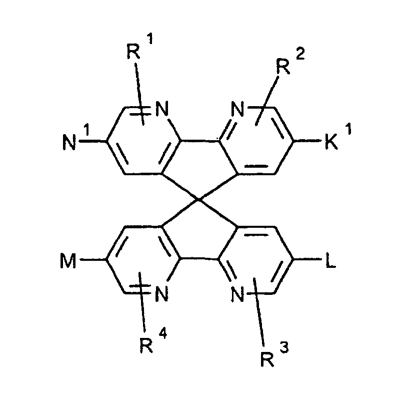

4. The use of an amorphous spiro compound of the formula (I), 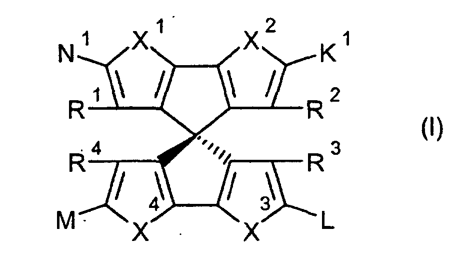 where the symbols have the following meanings:
X¹, X², X³, X⁴ are identical or different and are -S-, -O-, -NR⁵-, -CR⁵=N-, -CR⁵=CH-,
with the proviso that at least one of the groups X¹⁻⁴ is different from -CR⁵=CH-;
K¹, L, M, N¹, R¹, R², R³, R⁴ are identical or different and are each
a) hydrogen, -NO₂, -CN, -F or -Cl,
b) a straight-chain or branched alkyl radical having from 1 to 20 carbon atoms, where
b1) one or more nonadjacent CH₂ groups can be replaced by -O-, -S-, -CO-O-, -O-CO-, -O-CO-O-, NR⁵ or -Si(CH₃)₂- and/or
b2) one or more CH₂ groups can be replaced by - CH=CH-, -C≡C-, 1,4-phenylene, 1,4-cyclohexylene or 1,3-cyclopentylene and/or
b3) one or more H atoms can be replaced by F and/or Cl and/or
c) one of the following groups: 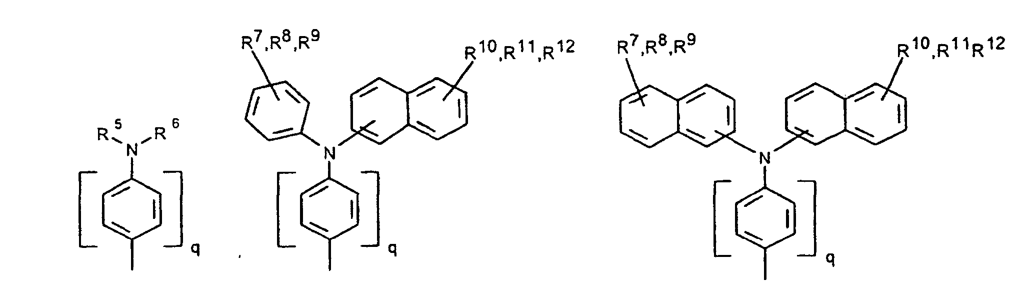 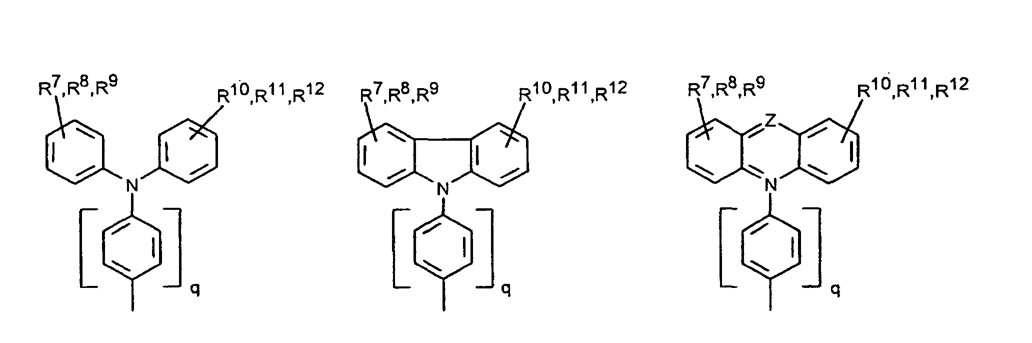 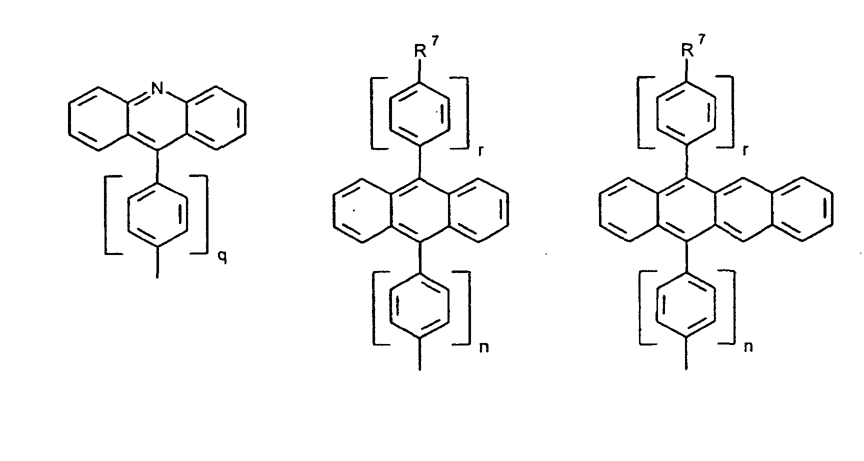 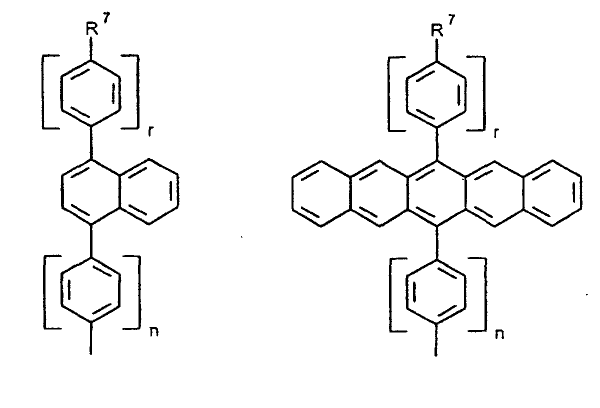 or 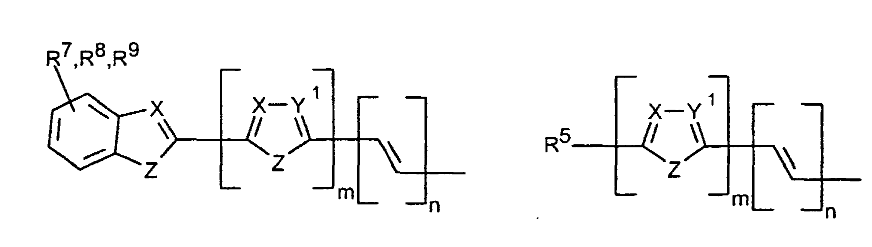
X, Y¹ are in each case identical or different and are =CR⁷- or =N-;
Z is -O-, -S-, -NR⁵-,
R⁵, R⁶ are in each case identical or different and are each
a) hydrogen
b) a straight-chain or branched alkyl radical having from 1 to 20 carbon atoms, where
b1) one or more nonadjacent CH₂ groups which are not bound to nitrogen can be replaced by -O-, -S-, -CO-O-, - O-CO-, -O-CO-O- or -Si(CH₃)₂ and/or
b2) one or more CH₂ groups can be replaced by -CH=CH-, -C≡C-, cyclopropane-1,2-diyl, 1,4-phenylene, 1,4-cyclohexylene or 1,3-cyclopentylene and/or
b3) one or more H atoms can be replaced by F and/or Cl and/or
b4) R⁵ and R⁶ together can also form a ring;
c) phenyl, biphenyl, 1-naphthyl, 2-naphthyl, 2-thienyl, 2-furanyl;
R⁷, R⁸, R⁹, R¹⁰, R¹¹, R¹² are identical or different and are each
a) hydrogen, -CN, -F, NO₂ or -Cl
b) a straight-chain or branched alkyl radical having from 1 to 20 carbon atoms, where
b1) one or more nonadjacent CH₂ groups can be replaced by -O-, -S-, -CO-O-, - O-CO-, -O-CO-O-, -NR⁵ or -Si(CH₃)₂- and/or
b2) one or more CH₂ groups can be replaced by -CH=CH-, -C≡C-, cyclopropane-1,2-diyl, 1,4-phenylene, 1,4-cyclohexylene or 1,3-cyclopentylene and/or
b3) one or more H atoms can be replaced by F and/or Cl;
c) phenyl, biphenyl, 1-naphthyl, 2-naphthyl, 2-thienyl, 2-furanyl, -O-phenyl, -O-biphenyl, -O-1-naphthyl, -O-2-naphthyl, -O-2-thienyl, -O-2-furanyl,
m, n, p, q, r are in each case identical or different and are 0, 1, 2, 3, 4, 5 or 6,
as charge transport material in photovoltaic cells.

5. A photovoltaic cell comprising a charge transport layer which comprises one or more spiro compounds as claimed in claim 4.

## Revendications

1. Composés spiraniques de formule générale (I) 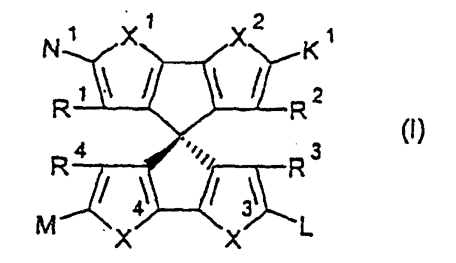 les symboles possédant les significations suivantes :
X¹, X², X³, X⁴ sont identiques ou différents et représentent un groupe -S-, -O-, -NR⁵-, -CR⁵=N-, -CR⁵=CH-,
à condition qu'au moins un des groupes X¹⁻⁴ soit différent de -CR⁵=N- ;
K¹, L, M, N¹, R¹, R², R³, R⁴ sont identiques ou différents et représentent
a) un atome d'hydrogène, un groupe -NO₂, -CN, -F ou -Cl,
b) un reste alkyle linéaire ou ramifié présentant 1 à 20 atomes de carbone,
b1) un ou plusieurs groupes CH₂ non adjacents pouvant être remplacés par des groupes -O-, -S-, -CO-O-, -O-CO-, -O-CO-O-, NR⁵ ou -Si(CH₃)₂- et/ou
b2) un ou plusieurs groupes CH₂ pouvant être remplacés par des groupes -CH=CH-, -C≡C-, 1,4-phénylène, 1,4-cyclohexylène ou 1,3-cyclopentylène et/ou
b3) un ou plusieurs atomes d'hydrogène pouvant être remplacés par des atomes de F et/ou Cl et/ou
c) un des groupes suivants 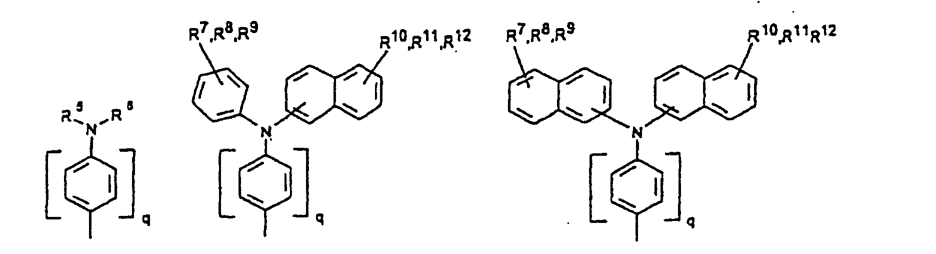 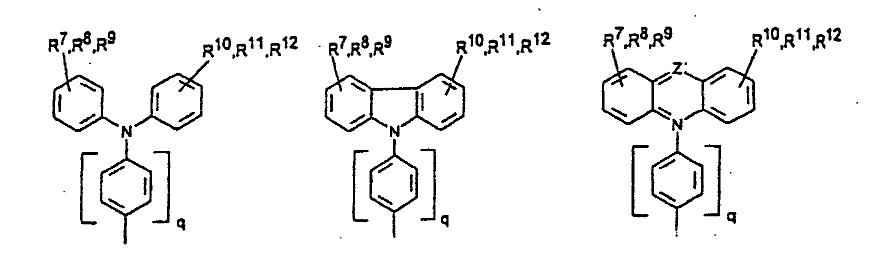 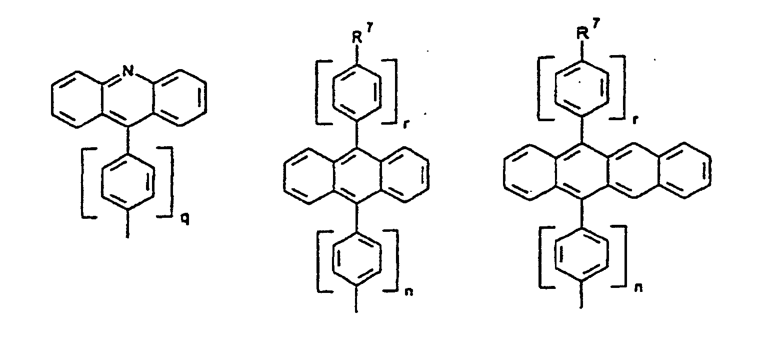 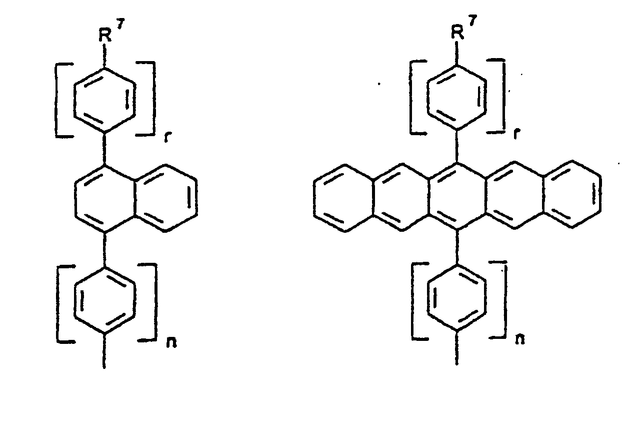 ou 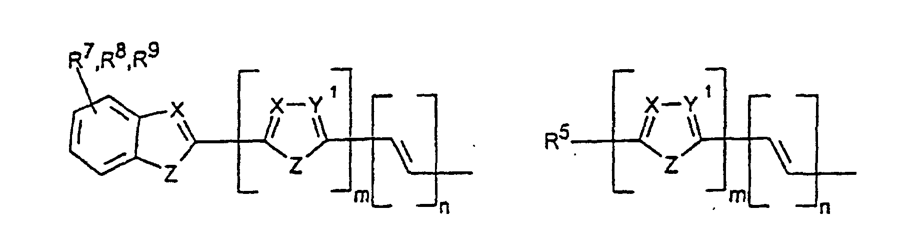 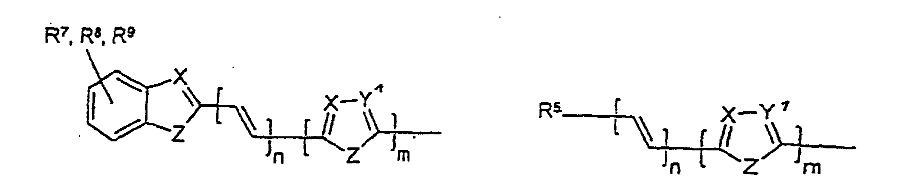
X, Y¹ représentent chacun, identiques ou différents un groupe =CR⁷- ou =N- ;
Z représente un groupe -O-, -S-, -NR⁵-,
R⁵, R⁶ représentent chacun, identiques ou différents,
a) un atome d'hydrogène,
b) un reste alkyle linéaire ou ramifié présentant 1 à 20 atomes de carbone,
b1) un ou plusieurs groupes CH₂ non adjacents et non liés à l'atome d'azote pouvant être remplacés par des groupes -O-, -S-, -CO-O-, -O-CO-, -O-CO-O- ou -Si(CH₃)₂- et/ou
b2) un ou plusieurs groupes CH₂ pouvant être remplacés par des groupes - CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène pouvant être remplacés par des atomes de F et/ou Cl et/ou
b4) R⁵ et R⁶ conjointement peuvent aussi former un cycle ;
c) représente un groupe phényle, biphényle, 1-naphtyle, 2-naphtyle, 2-thiényle, 2-furanyle ;
R⁷, R⁸, R⁹, R¹⁰, R¹¹, R¹² sont identiques ou différents et représentent
a) un atome d'hydrogène, un groupe -CN, -F, NO₂ ou -Cl,
b) un reste alkyle linéaire ou ramifié présentant 1 à 20 atomes de carbone,
b1) un ou plusieurs groupes CH₂ non adjacents pouvant être remplacés par des groupes -O-, -S-, -CO-O-, -O-CO-, -O-CO-O-, -NR⁵ ou -Si(CH₃)₂- et/ou
b2) un ou plusieurs groupes CH₂ pouvant être remplacés par des groupes - CH=CH-, -C≡C-, cyclopropane-1,2-diyle, 1,4-phénylène, 1,4-cyclohexylène ou 1,3-cycloopentylène et/ou
b3) un ou plusieurs atomes d'hydrogène pouvant être remplacés par des atomes de F et/ou Cl et/ou
c) représente un groupe phényle, biphényle, 1-naphtyle, 2-naphtyle, 2-thiényle, 2-furanyle, O-phényle, -O-biphényle, -O-1-naphtyle, -O-2-naphtyle, -O-2-thiényle, -O-2-furanyle,
m, n, p, q, r sont chaque fois identiques ou différents et valent 0, 1, 2, 3, 4, 5 ou 6,
à l'exception des composés de formule 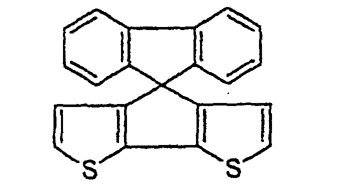 ainsi que du 4,5-diazospirobifluorène et du 4,4',5,5'-tétraazospirobifluorène.

2. Composés spiraniques selon la formule (I) de la revendication 1, **caractérisés en ce que**
a) X¹⁻⁴ sont identiques ou
b) X¹ est identique à X² et X³ est identique à X⁴.

3. Composés spiraniques selon la revendication 1 et/ou 2, pris dans le groupe
a) 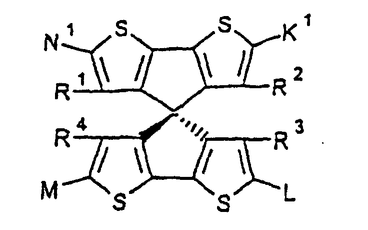
b) 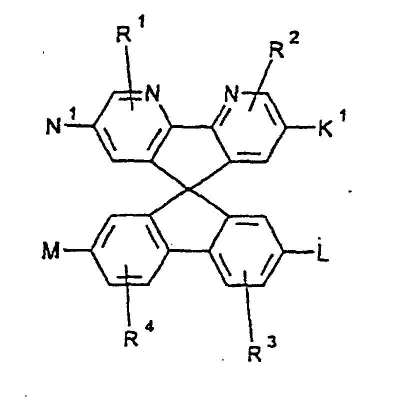
c)

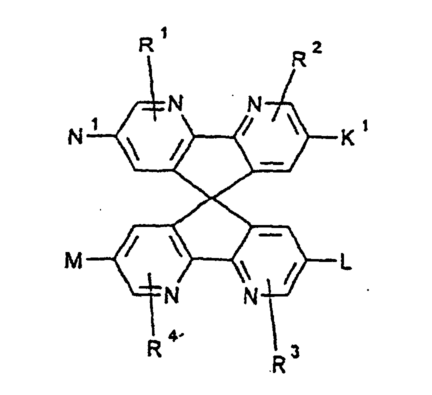

4. Utilisation d'un composé spiranique amorphe de formule générale (I) 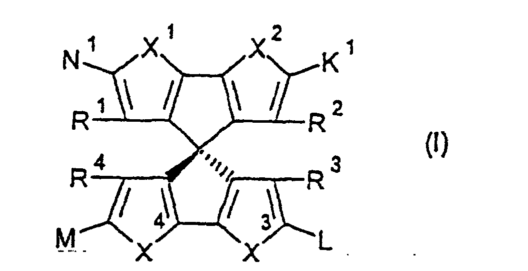 les symboles possédant les significations suivantes :
X¹, X², X³, X⁴ sont identiques ou différents et représentent un groupe -S-, -O-, -NR⁵-, -CR⁵=N-, -CR⁵=CH-, à condition qu'au moins un des groupes X¹⁻⁴ soit différent de -CR⁵=N- ;
K¹, L, M, N¹, R¹, R², R³, R⁴ sont identiques ou différents et représentent
a) un atome d'hydrogène, un groupe -NO₂, -CN, -F ou -Cl,
b) un reste alkyle linéaire ou ramifié présentant 1 à 20 atomes de carbone,
b1) un ou plusieurs groupes CH₂ non adjacents pouvant être remplacés par des groupes -O-, - S-, -CO-O-, -O-CO-, -O-CO-O-, -NR⁵ ou -Si(CH₃)₂- et/ou
b2) un ou plusieurs groupes CH₂ pouvant être remplacés par des groupes -CH=CH-, -C≡C-, 1,4-phénylène, 1,4-cyclohexylène ou 1,3-cyclopentylène et/ou
b3) un ou plusieurs atomes d'hydrogène pouvant être remplacés par des atomes de F et/ou Cl et/ou
c) un des groupes suivants 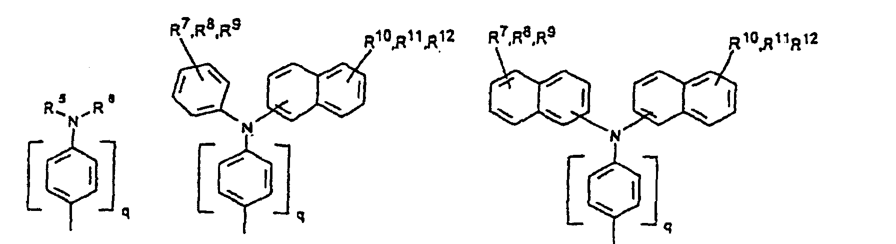 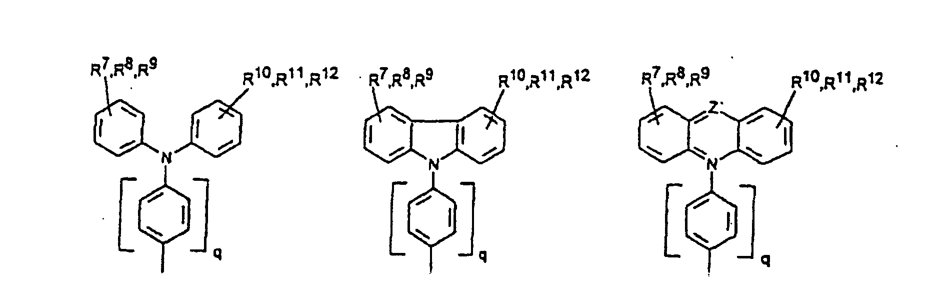 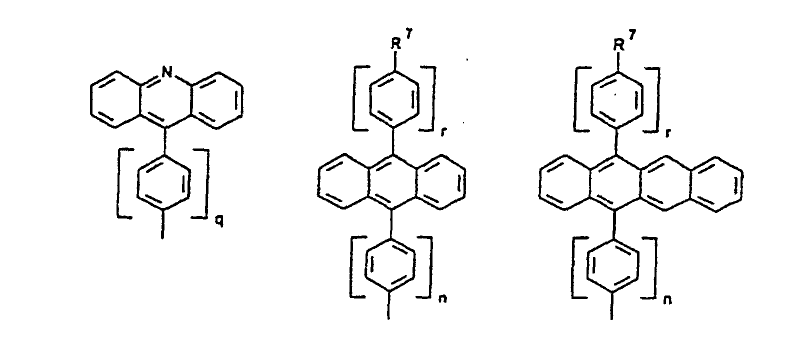 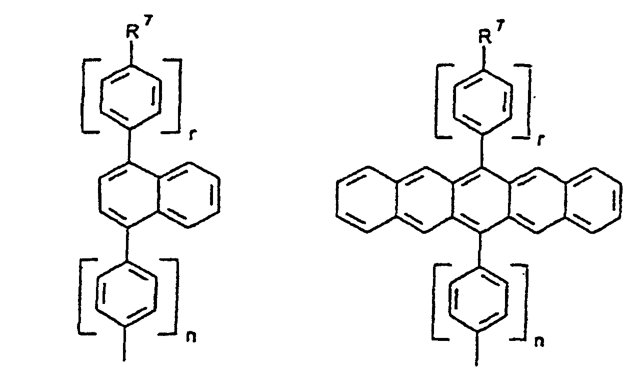 ou 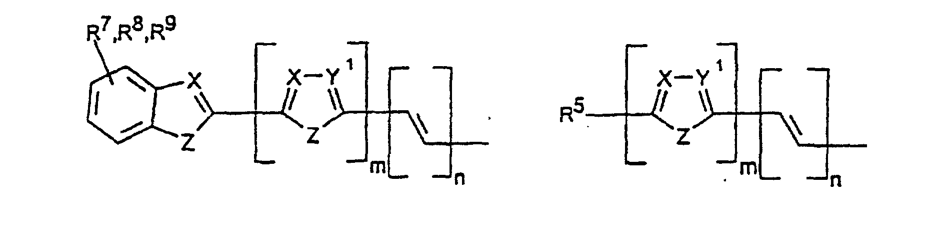 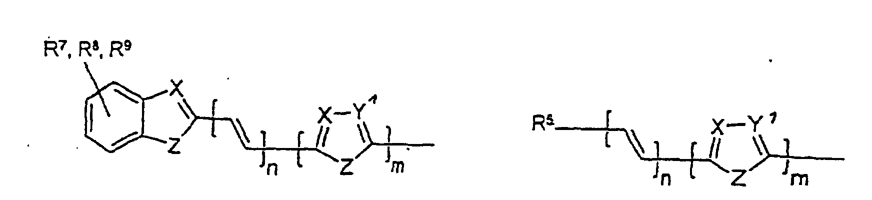
X, Y¹ représentent chacun, identiques ou différents, un groupe =CR⁷- ou =N- ;
Z représente un groupe -O-, -S-, -NR⁵-,
R⁵, R⁶ représentent chacun, identiques ou différents,
a) un atome d'hydrogène
b) un reste alkyle linéaire ou ramifié présentant 1 à 20 atomes de carbone,
b1) un ou plusieurs groupes CH₂ non adjacents et non liés à un atome d'azote pouvant être remplacés par des groupes -O-, -S-, -CO-O-, -O-CO-, -O-CO-O- ou -Si(CH₃)₂- et/ou
b2) un ou plusieurs groupes CH₂ pouvant être remplacés par des groupes - CH=CH-, -C≡C-, cyclopropane-1,2-diyle, 1,4-phénylène, 1,4-cyclohexylène ou 1,3-cycloopentylène et/ou
b3) un ou plusieurs atomes d'hydrogène pouvant être remplacés par des atomes de F et/ou Cl et/ou
b4) R⁵ et R⁶ conjointement peuvent aussi former un cycle ;
c) représente un groupe phényle, biphényle, 1-naphtyle, 2-naphtyle, 2-thiényle, 2-furanyle ;
R⁷, R⁸, R⁹, R¹⁰, R¹¹, R¹² sont identiques ou différents et représentent
a) un atome d'hydrogène, un groupe -CN, -F, NO₂ ou -Cl,
b) un reste alkyle linéaire ou ramifié présentant 1 à 20 atomes de carbone,
b1) un ou plusieurs groupes CH₂ non adjacents pouvant être remplacés par des groupes -O-, -S-, -CO-O-, -O-CO-, -O-CO-O-, -NR⁵ ou -Si(CH₃)₂- et/ou
b2) un ou plusieurs groupes CH₂ pouvant être remplacés par des groupes - CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène pouvant être remplacés par des atomes de F et/ou Cl et/ou
c) représente un groupe phényle, biphényle, 1-naphtyle, 2-naphtyle, 2-thiényle, 2-furanyle, -O-phényle, -O-biphényle, -O-1-naphtyle, -O-2-naphtyle, -O-2-thiényle, -O-2-furanyle,
m, n, p, q, r sont chaque fois identiques ou différents et valent 0, 1, 2, 3, 4, 5 ou 6,
en tant que matière de transport de charge dans des cellules photovoltaïques.

5. Cellule photovoltaïque renfermant une couche de transport de charge qui contient un ou plusieurs composés spiraniques selon la revendication 4.
